# EUROPEAN PATENT APPLICATION

(11) **EP 4 137 508 A1**
(43) Date of publication of application: **22.02.2023**
(21) Application number: 22151475.5
(22) Date of filing: 13.01.2022
(51) Int. Cl.: C07K 14/47, A61K 38/00, A61K 47/64, C07K 14/00

(54) **VARIANTS OF ANKYRIN REPEAT DOMAINS**

(30) Priority: 17.08.2021 WO PCT/EP2021/072819; 28.09.2021 EP 21199643
(71) Applicant: Athebio AG, 8952 Zurich-Schlieren (CH)
(72) Inventor: Forrer, Patrik, 8952 Zurich-Schlieren (CH); Eapen, Rohan Sakariah, 8952 Zurich-Schlieren (CH)

(57) **Abstract**

Described herein are proteins comprising an ankyrin repeat domain with a mutation in the internal ankyrin repeat that is adjacent to the N-terminal capping module, in particular a mutation at position 23 of said internal ankyrin repeat, as well as related products and methods.

## Description

### Field of the invention

The present invention relates to variants of an ankyrin repeat domain and related products and methods. In particular, the present invention relates to an ankyrin repeat domain having an amino acid residue of the leucine class at position 23 of the internal ankyrin repeat that is adjacent to the N-terminal capping module.

### Background of the invention

Different classes of specific binding proteins have evolved in nature, the most widely known class being immunoglobulins of vertebrates. Another class of specific binding proteins are repeat proteins. Similar to the role that immunoglobulins play in vertebrates, repeat proteins were found to be involved in the adaptive immune system of jawless fish. However, repeat proteins play a much wider role beyond this function and mediate protein-protein interactions across all phyla to fulfill diverse biological functions. In fact, they constitute the largest group of natural proteins mediating specific binding (e.g. reviewed in Forrer, P., et al., FEBS letters 539, 2-6, 2003). Repeat proteins bind their targets via the repeat domain, which is made up of a variable number of repeats that stack on each other through their conserved interfaces to create the compactly folded repeat domain. Specific target binding is then achieved through variable residues on the surface of the repeat domain (Forrer 2003, loc. cit. and WO 2002/020565).

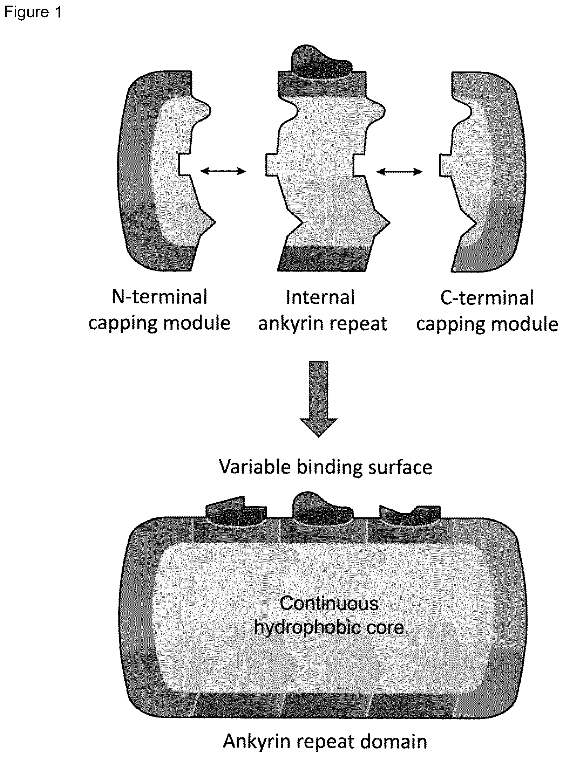

Ankyrin repeat proteins are a well-studied class of repeat proteins (e.g. Binz, H.K., et al., Nat. Biotechnol. 22, 575-582, 2004 and Mosavi, L.K., et al., Protein Sci. 2004 Jun;13(6):1435-48). The ankyrin repeat usually comprises 33 amino acid residues forming two antiparallel alpha-helices and a beta-turn. The folded ankyrin repeat domain comprising the stacked ankyrin repeats has a right-handed solenoid structure with a compact hydrophobic core and a large binding surface, which allows it to adapt to its respective binding partners. The terminal capping modules of the ankyrin repeat domain usually have a divergent sequence with polar residues to facilitate interaction with the solvent, thus capping the hydrophobic core. The basic architecture of the ankyrin repeat domain is shown in Figure 1.

Various attempts have been made to derive a consensus ankyrin repeat motif from naturally occurring ankyrin repeats as a basis for designing recombinant ankyrin repeat scaffolds.

Mosavi and colleagues originally derived a recombinant construct that only comprises consensus ankyrin repeats without specific capping modules (Mosavi, L.K., et al., Proc Natl Acad Sci USA. 2002 Dec 10;99(25):16029-34). To mitigate solubility issues, these constructs were later engineered to contain surface-exposed polar residues in the terminal consensus ankyrin repeats (Mosavi, L.K. and Peng, Z.Y., Protein Eng. 2003 Oct;16(10):739-45).

Aksel et al. derived their recombinant ankyrin repeat scaffold from a different consensus ankyrin repeat motif (Aksel, T., et al., Structure. 2011 Mar 9;19(3):349-60). Their scaffold also comprises capping repeats that closely match the consensus sequence but contain a few charged or polar residues to improve solubility.

Yet another consensus ankyrin repeat motif formed the basis for the recombinant ankyrin repeat scaffold from Plückthun and colleagues (e.g., Binz, H.K., et al., J. Mol. Biol., 332, 489-503, 2003 and WO 2002/020565). Unlike Mosavi et al. and Aksel et al. that took the consensus ankyrin repeat motif as a basis for their respective capping modules, Plückthun et al. derived the capping modules from the guanine-adenine-binding protein (GA-binding protein), a naturally occurring ankyrin repeat protein (PDB: 1AWC_B), which has a sequence that is largely diverging from the internal ankyrin repeats having the consensus sequence.

The design of Plückthun and colleagues has some further characteristics that allow the recombinant ankyrin repeat scaffold to be used as a versatile binding protein. In particular, fixed and variable positions were defined in the internal consensus ankyrin repeats (the latter also being referred to as randomized positions). The fixed positions correspond mainly to framework residues that are responsible for the structural integrity of the ankyrin repeat domain, including, for the interrepeat stacking interactions. The variable positions correspond to surface-exposed residues that do not strongly contribute to the structural integrity of the ankyrin repeat domain but are potentially involved in target binding (though surface-exposed framework residues may be involved in target binding too).

Through randomization of the variable positions in the internal consensus ankyrin repeats, libraries of proteins have been created, wherein each protein comprises an ankyrin repeat domain with different binding specificity (Binz, 2004, loc. cit.).

Using such library of recombinant ankyrin repeat proteins, ankyrin repeat proteins against specific targets can be selected with common selection methods, including phage display, ribosome display and yeast display, and were shown to have favorable properties. While displaying binding specificities and affinities that are comparable to immunoglobulins, such recombinant ankyrin repeat proteins are much more robust and can be easily engineered into multispecific binding proteins that are easily expressed and purified (e.g. reviewed in Plückthun, A., Annu. Rev. Pharmacol. Toxicol. 55, 489-511, 2015).

Though the recombinant ankyrin repeat scaffold originally conceived by Plückthun and colleagues was already stable, various mutations have been reported that yet further increase thermostability of such proteins. Interlandi et al. reported stabilizing mutations in the C-terminal capping module (Interlandi, G., et al., J Mol Biol. 2008 Jan 18;375(3):837-54). Similarly, stabilizing mutations in the N-terminal capping module have been reported (WO 2012/069655; PCT/EP2021/072819 and Schilling, J., et al., J Biol Chem. 2021 Nov 15;298(1):101403).

There remains a need to further improve the properties of proteins comprising an ankyrin repeat domain, such as the thermostability of the ankyrin repeat domain.

### Summary of the invention

The building blocks of the ankyrin repeat domain described by Binz et al. were engineered using two different approaches (Binz, 2003, loc. cit.). Whereas the internal ankyrin repeats were derived from a consensus design approach, its capping modules were derived from the GA-binding protein, a naturally occurring ankyrin repeat protein (PDB: 1AWC_B). In line with this, the interfaces between the internal ankyrin repeats, which are the direct result of the consensus design approach, may be regarded as optimized by nature, an optimization step that has not taken place for the interfaces between the capping modules and their respectively adjacent internal ankyrin repeat. The present inventors thus tried to optimize the interface between the N-terminal capping module and its adjacent internal ankyrin repeat and surprisingly found that mutating position 23 of the adjacent internal ankyrin repeat into an amino acid residue of the leucine class, such as leucine or isoleucine, results in an increased thermostability of the ankyrin repeat domain. Without wishing to be bound by theory, it is believed that such mutation improves the interaction between the N-terminal capping module and its neighboring internal ankyrin repeat, thus increasing the overall stability of the ankyrin repeat domain.

The present inventors surprisingly found that the above mutation even further increases thermostability of an ankyrin repeat domain that already contains mutations known to increase thermostability of the ankyrin repeat domain, such as I, T, A, V, L and M at position 15 of the N-terminal capping module (PCT/EP2021/072819) and I, V and L at position 22 of the N-terminal capping module (WO 2012/069655).

Accordingly, the present invention provides a protein comprising an ankyrin repeat domain, wherein the internal ankyrin repeat of the ankyrin repeat domain that is adjacent to the N-terminal capping module has an amino acid residue of the leucine class selected from L and I at position 23.

In further aspects, the present invention provides a protein library comprising such protein and a method of selection using such protein library.

The present invention also provides a nucleic acid encoding the protein of the invention and a vector or cell comprising such nucleic acid.

In a further aspect, the present invention provides a pharmaceutical composition comprising the protein of the invention, a nucleic acid encoding it or a vector or cell comprising a nucleic acid encoding the protein of the invention.

In a further aspect, the present invention provides a method of preparing a protein of the invention comprising culturing a cell having a nucleic acid encoding the protein under conditions allowing expression thereof and then purifying the expressed protein.

In a further aspect, the present invention relates to the protein of the invention for use in a method of treatment.

Related products and methods are also provided, as will be apparent from the following detailed description.

### Brief Description of the Figures

**Figure 1****:** The basic architecture of an ankyrin repeat domain. Multiple internal ankyrin repeats stack on each other to form a hydrophobic core, which gets shielded on both ends from the solvent by terminal capping modules. The variable surface residues allow the ankyrin repeat domain to bind to different targets.

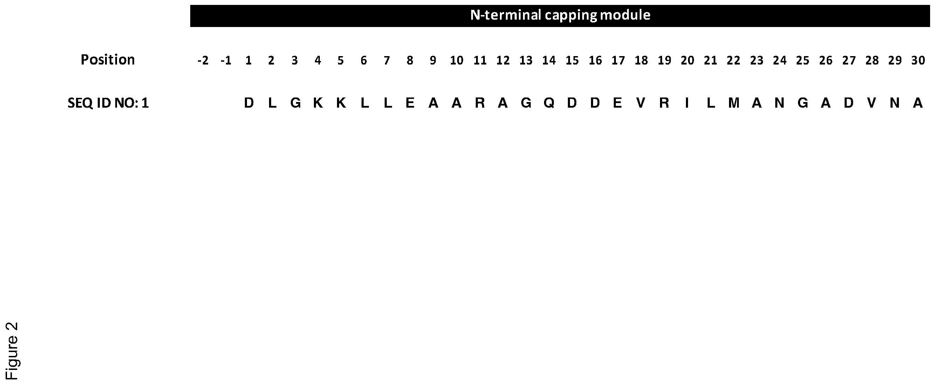

**Figure 2****:** The archetypal designed ankyrin repeat domain sequence of the N-terminal capping module as described by Binz et al. (Binz, 2003, loc. cit.). The sequence of the N-terminal capping module corresponds to SEQ ID NO: 1.

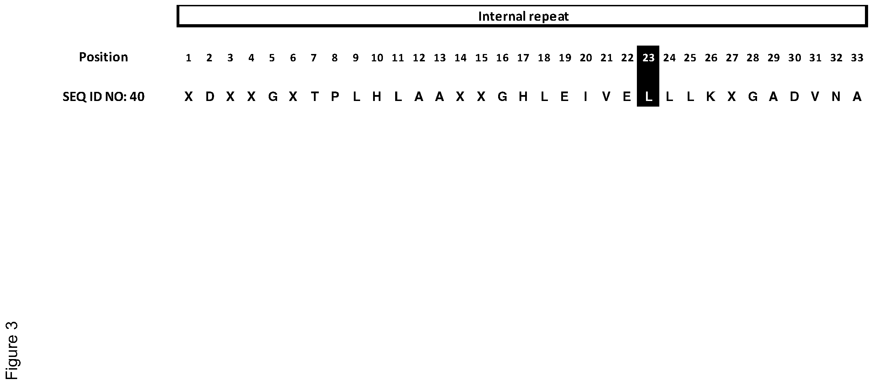

**Figure 3****:** The archetypal designed ankyrin repeat domain sequence of the internal ankyrin repeat as described by Binz et al. with an additional mutation at position 23 of the internal ankyrin repeat from V to L (Binz, 2003, loc. cit.). The sequence of the internal ankyrin repeat corresponds to SEQ ID NO: 40. Position 23 of the internal ankyrin repeat is highlighted.

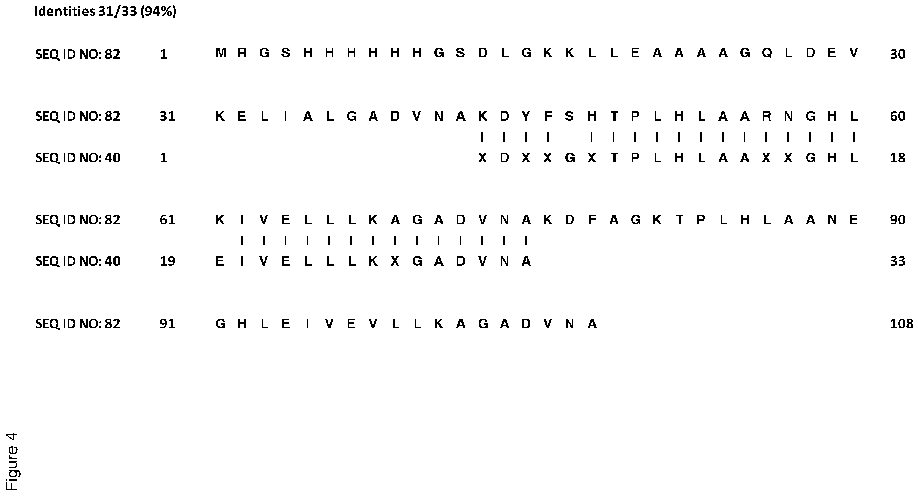

**Figure 4****:** Exemplary sequence alignment of SEQ ID NO: 40 and SEQ ID NO: 82. The positions of SEQ ID NO: 40 that are indicated with an "X" can be occupied by any amino acid residue and SEQ ID NO: 40 and SEQ ID NO: 82 therefore have 31 out of 33 identical amino acid residues across the alignment window (i.e. 94% sequence identity).

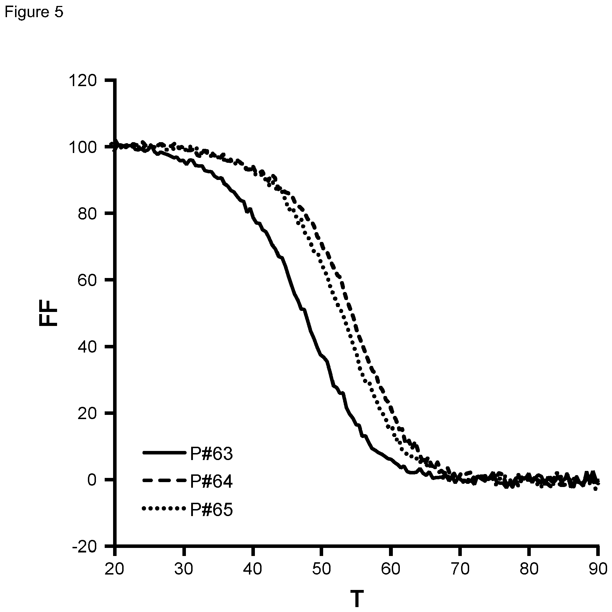

**Figure 5****:** Thermal stability of the ankyrin repeat domains P#63, P#64 and P#65, which have an identical sequence except for position 23 of the internal ankyrin repeat that is adjacent to the N-terminal capping module, which is occupied by V, I and L, respectively. Traces from thermal denaturation of P#63, P#64 and P#65 are shown. The Tm values for P#63, P#64 and P#65 were determined to be 48.0°C, 54.9°C and 53.4°C, respectively. FF, fraction folded in %; T, temperature in °C.

### Definitions

"A", "an", and "the" include plural reference unless the context clearly dictates otherwise. Thus, for example, reference to a protein comprising an ankyrin repeat domain refers to one or more such proteins.

The internal ankyrin repeat that is "adjacent" to the N-terminal capping module refers to the internal ankyrin repeat that is directly C-terminal of the N-terminal capping module forming an interface with the N-terminal capping module.

The amino acid residues are referred to herein interchangeably by their full name, their three-letter code or their one-letter code. The "naturally occurring amino acid residues" refer to the twenty amino acid residues that are most commonly found in nature, i.e. A, R, N, D, C, E, Q, G, H, I, L, K, M, F, P, S, T, W, Y and V.

An "ankyrin repeat" refers to a short sequence of amino acid residues forming a structural motif. Ankyrin repeats occur in consecutive copies, are involved in protein-protein interactions and the core of the ankyrin repeat forms a helix-loop-helix structure (e.g., SMART accession number: SM00248).

The term "ankyrin repeat domain" refers to a protein domain comprising an N-terminal capping module, a C-terminal capping module and one or more ankyrin repeats in between (also referred to as "internal ankyrin repeats"). The folded ankyrin repeat domain has a right-handed solenoid structure with a large binding surface that is adaptable to specifically bind targets. The ankyrin repeat domain is generally very robust and can sustain a significant number of mutations, including substitutions, additions and deletions, without destroying its overall structure or function. The residues that contribute to the structural integrity of the ankyrin repeat domain, including the interrepeat interactions, are referred to as "framework residues", whereas the residues that contribute to target binding, either through direct interaction with the target or by influencing residues that directly interact with the target, e.g., by stabilizing them, are referred to as "target interaction residues". A single amino acid residue can be both - a framework and a target interaction residue - at the same time and framework residues and target interaction residues may be found not only in the internal ankyrin repeats, but also the N-terminal capping module and/or the C-terminal capping module.

The internal ankyrin repeats contribute to the structural stability of the ankyrin repeat domain through their stacking interactions with the neighboring repeats. An internal ankyrin repeat usually consists of 33 amino acid residues.

The capping modules have a hydrophobic inside surface that is suitable for interacting with the adjacent internal ankyrin repeat and a hydrophilic outside surface to shield the hydrophobic core from the solvent. In some embodiments, the N-terminal capping module and/or the C-terminal capping module are a N-terminal capping repeat and/or C-terminal capping repeat, respectively, which have a similar or the same fold as the adjacent internal ankyrin repeat(s) and/or sequence similarities to said adjacent internal ankyrin repeat(s).

The terms "binding", "specific binding" or the like when used in reference to a target mean a binding interaction that is measurably different from a non-specific interaction, e.g., the interaction with a control molecule that is unrelated to the specific target. Control molecules that are commonly used to measure such non-specific interaction include bovine serum albumin, bovine casein and *Escherichia coli* (*E. coli*) maltose binding protein. In certain instances, the terms "binding", "specific binding" or the like mean that only the target is bound and substantially no other molecule. Specific binding can be determined, for instance, by measuring the dissociation constant (Kd) for the target and/or by comparing the binding to the target with the binding to a control molecule. The Kd can be measured by various conventional techniques, such as isothermal titration calorimetry, radioligand binding assay, fluorescence resonance energy transfer, and surface plasmon resonance. The binding specificity is generally measured in standardized solutions, such as PBS. For instance, the Kd for the target in PBS is at least 10, at least 10², at least 10³ or at least 10⁴ times lower than the corresponding Kd for a control molecule that is unrelated to the specific target.

The term "designed ankyrin repeat protein" or "DARPin" refers to a non-natural protein comprising an ankyrin repeat domain. In some embodiments, such a DARPin has a repeat sequence motif that was derived from natural ankyrin repeats, e.g. by consensus design (see, e.g., Forrer et al., 2004 Chem Bio Chem, 5, 2, 183-189 and Binz 2003, loc. cit).

The term "fraction of refolded ankyrin repeat domains after thermal denaturation" refers to the fraction of ankyrin repeat domains that refold into their native state after thermal denaturation.

The term "library" as used in reference to a protein or nucleic acid library refers to a collection of proteins and nucleic acids, respectively.

The term "melting temperature" or "Tm" refers to the temperature at which 50% of the protein is unfolded in a certain buffer, e.g. PBS.

The term "PBS" refers to phosphate-buffered saline. In some embodiments, PBS contains 137 mM NaCl, 10 mM phosphate and 2.7 mM KCI and has a pH of 7.4.

The term "percent (%) sequence identity" with respect to a reference amino acid sequence specified herein (e.g. the amino acid sequence of SEQ ID NO: 40) is defined as the percentage of amino acid residues in a candidate amino acid sequence that is identical with the amino acid residues in the reference amino acid sequence, after aligning the sequences and introducing gaps, if necessary, to achieve the maximum percent sequence identity, and not considering any conservative substitutions as part of the sequence identity. In some embodiments, such alignment comprises no gaps. Unless specified otherwise, the comparison window is the entire length of the reference amino acid sequence. Alignment for purposes of determining percent amino acid sequence identity can be achieved in various ways that are within the skill in the art, for instance, using publicly available computer software such as BLAST, BLAST-2, ALIGN or GenePAST. In some embodiments, the GenePAST algorithm, formerly known as KERR algorithm (Dufresne G, et al. Nat Biotechnol. 2002 Dec;20(12):1269-71), is used for alignment purposes. Those skilled in the art can determine appropriate parameters for measuring alignment, including any algorithms needed to achieve maximal alignment, in particular, over the full length of the reference amino acid sequence. Further examples of how to determine the percentage of sequence identity can be found in WO 2009/058564 A2, page 93, line 14 to page 102, line 5. When determining the sequence identity, it is understood that if an "X" in a reference amino acid sequence, such as in SEQ ID NO: 40, is further defined in the sequence listing as being selected from a certain group of amino acid residues, e.g. any amino acid residue, the "X" is counted as a match in a sequence alignment if the amino acid residue of the candidate sequence is identical to one of the amino acid residues defined for this position in the reference sequence. An exemplary sequence alignment reflecting this is shown in Figure 4.

The term "pharmaceutically acceptable carrier" refers to buffers, carriers, and other excipients suitable for use in contact with tissues of humans and/or animals without excessive toxicity, allergic response, irritation, or other problem or complication, commensurate with a reasonable benefit/risk ratio. The carrier(s) should be "acceptable" in the sense of being compatible with the other ingredients of the formulations and not deleterious to the recipient. Pharmaceutically acceptable carriers include buffers, solvents, dispersion media, coatings, isotonic and absorption delaying agents, and the like, that are compatible with pharmaceutical administration.

The term "pharmaceutical composition" refers to a composition comprising at least one active agent and at least one pharmaceutically acceptable carrier. A pharmaceutical composition is generally formulated and administered to exert a pharmaceutically useful effect while minimizing undesirable side effects.

The "position(s)" of the N-terminal capping module referred to herein may relate to the corresponding position(s) of SEQ ID NO: 1, which is the archetypal N-terminal capping module of designed ankyrin repeat proteins that remains commonly used in scientific studies (Binz, 2003, loc. cit.; also see Fig. 2). Accordingly, in some embodiments, the position(s) of the N-terminal capping module relate to the corresponding position(s) of SEQ ID NO: 1. In light of the high sequence similarity of SEQ ID NOs: 1 to 38, the respective positions of these sequences are well aligned and the position(s) of the N-terminal capping module referred to herein may similarly relate to the corresponding position(s) of one or more of SEQ ID NOs: 1 to 38. Accordingly, in some embodiments, the position(s) of the N-terminal capping module relate to the corresponding position(s) of any one of SEQ ID NOs: 1 to 38. In particular, in embodiments further defining the sequence of the N-terminal capping module by way of reference to one or more of SEQ ID NOs: 1 to 38, the position(s) of the N-terminal capping module may relate to the corresponding position(s) of the respective one or more of SEQ ID NOs: 1 to 38 used to further define the sequence of the N-terminal capping module. For instance, for a protein comprising an ankyrin repeat domain having an N-terminal capping module with I at position 15 and comprising a sequence with at least 70% sequence identity to one or more of SEQ ID NOs: 10 to 37, position 15 may refer to the corresponding position of SEQ ID NO: 1, which is D in SEQ ID NO: 1, or it may refer to the corresponding position of any one of SEQ ID NOs: 10 to 37. The "position(s)" of the internal ankyrin repeat that is adjacent to the N-terminal capping module referred to herein may relate to the corresponding position(s) of SEQ ID NO: 40, which is, apart from the mutation at position 23 from V to L, the archetypal internal ankyrin repeat of designed ankyrin repeat proteins that remains commonly used in scientific studies (Binz, 2003, loc. cit.; also see Fig. 3). Accordingly, in some embodiments, the position(s) of the internal ankyrin repeat that is adjacent to the N-terminal capping module relate to the corresponding position(s) of SEQ ID NO: 40. In light of the high sequence similarity of SEQ ID NOs: 39 to 46, the respective positions of these sequences are well aligned and the position(s) of the internal ankyrin repeat that is adjacent to the N-terminal capping module referred to herein may similarly relate to the corresponding position(s) of one or more of SEQ ID NOs: 39 to 46. Accordingly, in some embodiments, the position(s) of the internal ankyrin repeat that is adjacent to the N-terminal capping module relate to the corresponding position(s) of any one of SEQ ID NOs: 39 to 46. In particular, in embodiments further defining the sequence of the internal ankyrin repeat that is adjacent to the N-terminal capping module by way of reference to one or more of SEQ ID NOs: 39 to 46, the position(s) of the internal ankyrin repeat that is adjacent to the N-terminal capping module may relate to the corresponding position(s) of the respective one or more of SEQ ID NOs: 39 to 46 used to further define the sequence of the internal ankyrin repeat that is adjacent to the N-terminal capping module. For instance, for a protein comprising an ankyrin repeat domain having an internal ankyrin repeat that is adjacent to the N-terminal capping module with L at position 23 and comprising a sequence with at least 70% sequence identity to one or more of SEQ ID NOs: 42 to 44, position 23 may refer to the corresponding position of SEQ ID NO: 40 or it may refer to the corresponding position of any one of SEQ ID NOs: 42 to 44. In some embodiments, the "position(s)" of the N-terminal capping module refer to the corresponding position(s) of SEQ ID NO: 1 and the "position(s)" of the internal ankyrin repeat that is adjacent to the N-terminal capping module refer to the corresponding position(s) of SEQ ID NO: 40. Furthermore, "corresponding" in this context means that the respective positions align in a sequence alignment. Alignment for purposes of determining which amino acid residue corresponds to which position of a specific sequence can be achieved in various ways, as is further described above.

The term "recombinant", as used in reference to a protein, refers to a protein produced from a recombinant nucleic acid. A "recombinant nucleic acid" refers to a nucleic acid molecule formed by laboratory methods of genetic recombination or gene synthesis.

The term "target", as used, for instance, in conjunction with the specific binding property of an ankyrin repeat domain, refers to any substance or structure. It may refer to a single molecule, such as a protein, peptide, small-molecule or sugar, as well as complexed molecules, such as interacting proteins or proteins binding to non-proteinaceous compounds. It may also refer to more macromolecular structures, such as cells, tissues, viruses or bacteria.

The terms "treating" or "treatment" of a disease, condition or symptom refers to obtaining therapeutic and/or prophylactic benefit, including alleviating, ablating, ameliorating, or preventing a disease, condition or symptom, preventing additional symptoms, ameliorating or preventing the underlying metabolic causes of symptoms, inhibiting the disease or condition, e.g., arresting or slowing down the development of the disease or condition, relieving the disease or condition, causing regression of the disease or condition, relieving a condition caused by the disease or condition, or stopping the symptoms of the disease or condition.

### Detailed description of the invention

The protein of the invention comprises an ankyrin repeat domain that has an amino acid residue of the leucine class selected from L and I at position 23 of the internal ankyrin repeat that is adjacent to the N-terminal capping module. In some embodiments, the amino acid residue at position 23 of the internal ankyrin repeat that is adjacent to the N-terminal capping module is L. In some embodiments, the amino acid residue at position 23 of the internal ankyrin repeat that is adjacent to the N-terminal capping module is I.

In some embodiments, the ankyrin repeat domain of the protein of the invention has improved properties, which may include improved thermostability, improved storage stability, improved thermodynamic stability (defined as the difference in free energy between the folded and unfolded states), improved folding and/or refolding properties (such as a higher fraction of refolded ankyrin repeat domains after thermal denaturation), reduced aggregation propensity and lower *in vivo* immunogenicity risk. Thus, in some embodiments, the protein of the invention comprises an ankyrin repeat domain that has an amino acid residue of the leucine class selected from L and I at position 23 of the internal ankyrin repeat that is adjacent to the N-terminal capping module and an improved property, such as an improved thermostability, as compared to a reference ankyrin repeat domain having the same sequence except for said position 23, which is, e.g., V in the reference ankyrin repeat domain.

In some embodiments, the ankyrin repeat domain has further mutations apart from the mutation at position 23 of the internal ankyrin repeat that is adjacent to the N-terminal capping module.

In some embodiments, the ankyrin repeat domain has a mutation in the N-terminal capping module that is selected from the following amino acid residues:

**Table 1:**

| **Position** | **Amino acid residue** |
|---|---|
| 1 | A, E, N, Q, G, S, T, K, D, R, H, C |
| 2 | E, L, Q, M, K, R, C |
| 3 | G, D, S, A, C |
| 4 | A, R, T, S, N, Q, K, D, E, H, C |
| 5 | A, R, T, S, N, Q, K, D, E, H, C |
| 6 | A, L, N, S, D, C |
| 7 | L, I, V, A, N, T, S, D, C |
| 8 | E, D, Q, A, N, S, T, K, R, H, C |
| 9 | A |
| 10 | V, S, A, C |
| 11 | L, S, Q, K, R, A, H, D, E, C, T, N, F, W, Y |
| 12 | K, R, A, T, S, N, Q, D, E, H, C |
| 13 | G, C |
| 14 | N, S, T, A, D, E, K, Q, R, H, C |
| 15 | M, I, T, A, L, V, S, N, D, Q, K, R, E, C |
| 16 | D, A, N, Q, S, T, K, E, R, H, C |
| 17 | D, A, N, Q, S, T, K, E, R, H, C |
| 18 | T, A, S, I, L, V, C |
| 19 | R, E, D, K, A, N, Q, S, T, H, C |
| 20 | N, K, R, T, S, E, Q, A, D, H, C, I, V |
| 21 | N, S, L, A, C |
| 22 | I, A, V, M, T, L, S, N, C |
| 23 | R, S, Q, K, N, A, E, D, H, C |
| 24 | A, H, K, R, L, I, V, C, G |
| 25 | G |
| 26 | A |
| 27 | N, D, C |
| 28 | T, V, S, P, A, C |
| 29 | D, N, C |
| 30 | A, C |

In some embodiments, the N-terminal capping module has an amino acid residue of Table 1 in one or more position(s). In some embodiments, the amino acid residue at one or more position(s) of the N-terminal capping module is selected from the group consisting of the amino acid residues shown for the respective position(s) in Table 1.

In some embodiments, the N-terminal capping module has an amino acid residue selected from the group consisting of L, S, Q, K, R, A, H, D and E at position 11. In some embodiments, the N-terminal capping module has an amino acid residue selected from the group consisting of K, E, Q, A and L at position 11. In some embodiments, the N-terminal capping module has an amino acid residue selected from the group consisting of K, E, A and L at position 11. In some embodiments, the N-terminal capping module has E or A at position 11. In some embodiments, the N-terminal capping module has A at position 11. In some embodiments, the N-terminal capping module has E at position 11.

In some embodiments, the N-terminal capping module has an amino acid residue selected from the group consisting of I, T, A, V, L and M at position 15. In particular, L, I and V at position 15 of the N-terminal capping module were found to combine well with the mutation at position 23 of the internal ankyrin repeat that is adjacent to the N-terminal capping module.

Usually, an ankyrin repeat domain having I or V at position 15 of the N-terminal capping module is similarly or less stable than an ankyrin repeat domain having L at position 15 of the N-terminal capping module. It was surprisingly found that in an ankyrin repeat domain having an amino acid residue of the leucine class at position 23 of the internal ankyrin repeat that is adjacent to the N-terminal capping module, the further combination with an I or V at position 15 of the N-terminal capping module resulted in an ankyrin repeat domain that is generally more stable than the same ankyrin repeat domain having L at position 15 of the N-terminal capping module. In particular, the combination of L at position 23 of the internal ankyrin repeat that is adjacent to the N-terminal capping module with I at position 15 of the N-terminal capping module resulted in the most stable ankyrin repeat domains (see Examples 1 and 2).

In some embodiments, the N-terminal capping module has an amino acid residue selected from the group consisting of I, V and L at position 15. In some embodiments, the N-terminal capping module has I or V at position 15. In some embodiments, the N-terminal capping module has I at position 15. In some embodiments, the N-terminal capping module has V at position 15. In some embodiments, the N-terminal capping module has L at position 15.

In some embodiments, the internal ankyrin repeat that is adjacent to the N-terminal capping module has L at position 23 and the N-terminal capping module has I at position 15. In some embodiments, the internal ankyrin repeat that is adjacent to the N-terminal capping module has L at position 23 and the N-terminal capping module has L at position 15. In some embodiments, the internal ankyrin repeat that is adjacent to the N-terminal capping module has L at position 23 and the N-terminal capping module has V at position 15. In some embodiments, the internal ankyrin repeat that is adjacent to the N-terminal capping module has L at position 23 and the N-terminal capping module has T at position 15. In some embodiments, the internal ankyrin repeat that is adjacent to the N-terminal capping module has L at position 23 and the N-terminal capping module has A at position 15. In some embodiments, the internal ankyrin repeat that is adjacent to the N-terminal capping module has L at position 23 and the N-terminal capping module has M at position 15. In some embodiments, the internal ankyrin repeat that is adjacent to the N-terminal capping module has I at position 23 and the N-terminal capping module has I at position 15. In some embodiments, the internal ankyrin repeat that is adjacent to the N-terminal capping module has I at position 23 and the N-terminal capping module has L at position 15. In some embodiments, the internal ankyrin repeat that is adjacent to the N-terminal capping module has I at position 23 and the N-terminal capping module has V at position 15. In some embodiments, the internal ankyrin repeat that is adjacent to the N-terminal capping module has I at position 23 and the N-terminal capping module has T at position 15. In some embodiments, the internal ankyrin repeat that is adjacent to the N-terminal capping module has I at position 23 and the N-terminal capping module has A at position 15. In some embodiments, the internal ankyrin repeat that is adjacent to the N-terminal capping module has I at position 23 and the N-terminal capping module has M at position 15.

In some embodiments, the N-terminal capping module has an amino acid residue selected from the group consisting of R, E, D, K, A, N, Q, S, T, H and C at position 19. In some embodiments, the N-terminal capping module has R at position 19. In some embodiments, the N-terminal capping module has K at position 19.

In some embodiments, the N-terminal capping module has L at position 2. In some embodiments, the N-terminal capping module has L at position 21. In some embodiments, the N-terminal capping module has L at position 2 and L at position 21.

In some embodiments, the N-terminal capping module has an amino acid residue selected from the group consisting of L, V, I and A at position 22. In some embodiments, the N-terminal capping module has an amino acid residue selected from the group consisting of L, V and I at position 22. In some embodiments, the N-terminal capping module has L at position 22. In some embodiments, the N-terminal capping module has V at position 22. In some embodiments, the N-terminal capping module has I at position 22. In some embodiments, the N-terminal capping module has A at position 22.

In some embodiments, the internal ankyrin repeat that is adjacent to the N-terminal capping module has L at position 23 and the N-terminal capping module has I at position 22. In some embodiments, the internal ankyrin repeat that is adjacent to the N-terminal capping module has L at position 23 and the N-terminal capping module has L at position 22. In some embodiments, the internal ankyrin repeat that is adjacent to the N-terminal capping module has L at position 23 and the N-terminal capping module has V at position 22. In some embodiments, the internal ankyrin repeat that is adjacent to the N-terminal capping module has I at position 23 and the N-terminal capping module has I at position 22. In some embodiments, the internal ankyrin repeat that is adjacent to the N-terminal capping module has I at position 23 and the N-terminal capping module has L at position 22. In some embodiments, the internal ankyrin repeat that is adjacent to the N-terminal capping module has I at position 23 and the N-terminal capping module has V at position 22.

In some embodiments, the N-terminal capping module has an amino acid residue selected from the group consisting of I, T, A, V, L and M at position 15 and an amino acid residue selected from the group consisting of L, V and I at position 22.

In some embodiments, the N-terminal capping module has L at position 15 and I at position 22. In some embodiments, the N-terminal capping module has M at position 15 and I at position 22. In some embodiments, the N-terminal capping module has T at position 15 and I at position 22. In some embodiments, the N-terminal capping module has I at position 15 and I at position 22. In some embodiments, the N-terminal capping module has A at position 15 and I at position 22. In some embodiments, the N-terminal capping module has V at position 15 and I at position 22.

In some embodiments, the N-terminal capping module has L at position 15 and L at position 22. In some embodiments, the N-terminal capping module has M at position 15 and L at position 22. In some embodiments, the N-terminal capping module has T at position 15 and L at position 22. In some embodiments, the N-terminal capping module has I at position 15 and L at position 22. In some embodiments, the N-terminal capping module has A at position 15 and L at position 22. In some embodiments, the N-terminal capping module has V at position 15 and L at position 22.

In some embodiments, the N-terminal capping module has L at position 15 and V at position 22. In some embodiments, the N-terminal capping module has M at position 15 and V at position 22. In some embodiments, the N-terminal capping module has T at position 15 and V at position 22. In some embodiments, the N-terminal capping module has I at position 15 and V at position 22. In some embodiments, the N-terminal capping module has A at position 15 and V at position 22. In some embodiments, the N-terminal capping module has V at position 15 and V at position 22.

In some embodiments, the N-terminal capping module has an amino acid residue selected from the group consisting of R, S, Q, K, N, A, E, D, H, C at position 23. In some embodiments, the N-terminal capping module has E at position 23. In some embodiments, the N-terminal capping module has A at position 23. In some embodiments, the N-terminal capping module has K at position 23.

In some embodiments, the N-terminal capping module has an amino acid residue selected from the group consisting of I, T, A, V, L and M at position 15, an amino acid residue selected from the group consisting of R and K at position 19, and an amino acid residue selected from the group consisting of L, V and I at position 22. In some embodiments, the N-terminal capping module has an amino acid residue selected from the group consisting of I, T, A, V, L and M at position 15, the amino acid residue R at position 19 and an amino acid residue selected from the group consisting of L, V and I at position 22. In some embodiments, the N-terminal capping module has an amino acid residue selected from the group consisting of I, T, A, V, L and M at position 15, the amino acid residue K at position 19 and an amino acid residue selected from the group consisting of L, V and I at position 22.

In some embodiments, the N-terminal capping module has an amino acid residue selected from the group consisting of I, T, A, V, L and M at position 15, an amino acid residue selected from the group consisting of L, V and I at position 22, and an amino acid residue selected from the group consisting of A and K at position 23. In some embodiments, the N-terminal capping module has an amino acid residue selected from the group consisting of I, T, A, V, L and M at position 15, an amino acid residue selected from the group consisting of L, V and I at position 22, and the amino acid residue A at position 23. In some embodiments, the N-terminal capping module has an amino acid residue selected from the group consisting of I, T, A, V, L and M at position 15, an amino acid residue selected from the group consisting of L, V and I at position 22, and the amino acid residue K at position 23.

In some embodiments, the N-terminal capping module has an amino acid residue selected from the group consisting of I, T, A, V, L and M at position 15, an amino acid residue selected from the group consisting of R and K at position 19, an amino acid residue selected from the group consisting of L, V and I at position 22, and an amino acid residue selected from the group consisting of A and K at position 23. In some embodiments, the N-terminal capping module has an amino acid residue selected from the group consisting of I, T, A, V, L and M at position 15, the amino acid residue R at position 19, an amino acid residue selected from the group consisting of L, V and I at position 22, and the amino acid residue K at position 23. In some embodiments, the N-terminal capping module has an amino acid residue selected from the group consisting of I, T, A, V, L and M at position 15, the amino acid residue K at position 19, an amino acid residue selected from the group consisting of L, V and at position 22 I, and the amino acid residue K at position 23. In some embodiments, the N-terminal capping module has an amino acid residue selected from the group consisting of I, T, A, V, L and M at position 15, the amino acid residue R at position 19, an amino acid residue selected from the group consisting of L, V and I at position 22, and the amino acid residue A at position 23. In some embodiments, the N-terminal capping module has an amino acid residue selected from the group consisting of I, T, A, V, L and M at position 15, the amino acid residue K at position 19, an amino acid residue selected from the group consisting of L, V and I at position 22, and the amino acid residue A at position 23.

In some embodiments, the N-terminal capping module has an amino acid residue selected from the group consisting of E and A at position 11, an amino acid residue selected from the group consisting of I, T, A, V, L and M at position 15, an amino acid residue selected from the group consisting of R and K at position 19, an amino acid residue selected from the group consisting of L, V and I at position 22, and an amino acid residue selected from the group consisting of A and K at position 23.

In some embodiments, the N-terminal capping module has an amino acid residue selected from the group consisting of R and K at position 19 and an amino acid residue selected from the group consisting of A and K at position 23. In some embodiments, the N-terminal capping module has R at position 19 and A at position 23. In some embodiments, the N-terminal capping module has K at position 19 and A at position 23. In some embodiments, the N-terminal capping module has R at position 19 and K at position 23. In some embodiments, the N-terminal capping module has K at position 19 and K at position 23.

In some embodiments, the N-terminal capping module has L at position 24. In some embodiments, the internal ankyrin repeat that is adjacent to the N-terminal capping module has L at position 23 and the N-terminal capping module has L at position 24. In some embodiments, the internal ankyrin repeat that is adjacent to the N-terminal capping module has I at position 23 and the N-terminal capping module has L at position 24. In some embodiments, the N-terminal capping module has an amino acid residue selected from the group consisting of I, T, A, V, L and M at position 15 and L at position 24. In some embodiments, the N-terminal capping module has an amino acid residue selected from the group consisting of L, V and I at position 22 and L at position 24. In some embodiments, the N-terminal capping module has an amino acid residue selected from the group consisting of I, T, A, V, L and M at position 15, an amino acid residue selected from the group consisting of L, V and I at position 22 and L at position 24.

In some embodiments, the N-terminal capping module has the amino acid sequence (R/K)(I/E)L(L/I/M)(A/K)(A/L) at positions 19 to 24, wherein the amino acid residue at the positions 19, 20, 22, 23 and 24 is selected from the group consisting of the amino acid residues shown in the respective parentheses. In some embodiments, the N-terminal capping module has one of the amino acid residues indicated for the respective positions in Table 1 at positions 19 to 24. In some embodiments, the N-terminal capping module has an amino acid sequence at positions 19 to 24 selected from the group consisting of: RELLKA, RILLKA, RILMAL, RELLKL, RILLKL, RELIKL, RILIKL, RELLAL, RILLAL, RELIAL, RILIAL, KILMAL, KELLKL, KILLKL, KELIKL, KILIKL, KELLAL, KILLAL, KELIAL and KILIAL. In some embodiments, the N-terminal capping module has the amino acid sequence KELIAL at positions 19 to 24.

In some embodiments, the N-terminal capping module does not comprise the amino acid sequence TPLH.

In some embodiments, the ankyrin repeat domain of the protein of the invention has an improved thermostability, such as a higher melting temperature and/or a higher fraction of refolded ankyrin repeat domains after thermal denaturation, as compared to a reference ankyrin repeat domain having the same amino acid sequence except for one or more of the mutations specified herein, for instance, the mutation at position 23 of the internal ankyrin repeat that is adjacent to the N-terminal capping module. In some embodiments having one or more of the mutation(s) in the N-terminal capping module in addition to the mutation at position 23 of the internal ankyrin repeat that is adjacent to the N-terminal capping module, the ankyrin repeat domain of the protein of the invention has an improved thermostability, such as a higher melting temperature and/or a higher fraction of refolded ankyrin repeat domains after thermal denaturation, as compared to a reference ankyrin repeat domain having the same amino acid sequence except for the mutation at position 23 of the internal ankyrin repeat that is adjacent to the N-terminal capping module and/or as compared to a reference ankyrin repeat domain having the same amino acid sequence except for the one or more mutation(s) in the N-terminal capping module.

In some embodiments, the reference ankyrin repeat domain not having the mutation at position 23 of the internal ankyrin repeat that is adjacent to the N-terminal capping module has an amino acid residue selected from the naturally occurring amino acid residues other than L and I at this position, such as V. In some embodiments, the reference ankyrin repeat domain not having one or more of the mutation(s) in the N-terminal capping module has an amino acid residue found in the corresponding position(s) of SEQ ID NO: 1 or SEQ ID NO: 2. For instance, in case of a mutation at position 15 of the N-terminal capping module, the amino acid residue at corresponding position 15 of the reference ankyrin repeat domain can be D. Similarly, in case of a mutation at position 22 of the N-terminal capping module, the amino acid residue at corresponding position 22 of the reference ankyrin repeat domain can be M. Similarly, in case of a mutation at position 24 of the N-terminal capping module, the amino acid residue at corresponding position 24 of the reference ankyrin repeat domain can be A.

In some embodiments, the ankyrin repeat domain of the protein of the invention (with or without additional mutations in the N-terminal capping module) has an improved thermostability, such as a higher melting temperature and/or a higher fraction of refolded ankyrin repeat domains after thermal denaturation, as compared to a reference ankyrin repeat domain having the same amino acid sequence except for position 23 of the internal ankyrin repeat that is adjacent to the N-terminal capping module, which is V in the reference ankyrin repeat domain. In some embodiments, the ankyrin repeat domain of the protein of the invention additionally has a mutation at position 15 of the N-terminal capping module and the ankyrin repeat domain has an improved thermostability, such as a higher melting temperature and/or a higher fraction of refolded ankyrin repeat domains after thermal denaturation, as compared to a first reference ankyrin repeat domain having the same amino acid sequence except for the mutation at position 23 of the internal ankyrin repeat that is adjacent to the N-terminal capping module, which is V in the first reference ankyrin repeat domain, and/or as compared to a second reference ankyrin repeat domain having the same amino acid sequence except for position 15 of the N-terminal capping module, which is D in the second reference ankyrin repeat domain. In some embodiments, the ankyrin repeat domain of the protein of the invention additionally has a mutation at position 22 of the N-terminal capping module and the ankyrin repeat domain has an improved thermostability, such as a higher melting temperature and/or a higher fraction of refolded ankyrin repeat domains after thermal denaturation, as compared to a first reference ankyrin repeat domain having the same amino acid sequence except for the mutation at position 23 of the internal ankyrin repeat that is adjacent to the N-terminal capping module, which is V in the first reference ankyrin repeat domain, and/or as compared to a second reference ankyrin repeat domain having the same amino acid sequence except for position 22 of the N-terminal capping module, which is M in the second reference ankyrin repeat domain. In some embodiments, the ankyrin repeat domain of the protein of the invention additionally has a mutation at position 24 of the N-terminal capping module and the ankyrin repeat domain has an improved thermostability, such as a higher melting temperature and/or a higher fraction of refolded ankyrin repeat domains after thermal denaturation, as compared to a first reference ankyrin repeat domain having the same amino acid sequence except for the mutation at position 23 of the internal ankyrin repeat that is adjacent to the N-terminal capping module, which is V in the first reference ankyrin repeat domain, and/or as compared to a second reference ankyrin repeat domain having the same amino acid sequence except for position 24 of the N-terminal capping module, which is A in the second reference ankyrin repeat domain.

In some embodiments, the ankyrin repeat domain of the protein of the invention additionally has one or more mutation(s) in the N-terminal capping module and the mutation at position 23 of the internal ankyrin repeat that is adjacent to the N-terminal capping module and the one or more mutation(s) in the N-terminal capping module at least additively increase thermostability of the ankyrin repeat domain. Such at least additively increased thermostability may be reflected, for instance, by an at least additively increased melting temperature or an at least additively increased fraction of refolded ankyrin repeat domains after thermal denaturation.

Methods for measuring the thermostability of a protein or a protein domain are well-known to the person skilled in the art. For instance, the thermostability can be measured by a thermal shift assay, differential scanning calorimetry and circular dichroism (CD). Another possible approach is to use differential scanning fluorimetry (e.g. Nielsen et al., 2007, Nat Protoc. 2, 9:2212-21). In this method, unfolding of the protein is measured with a fluorescent dye that binds to hydrophobic parts of the protein. As the protein unfolds, more hydrophobic parts become exposed causing an increase in fluorescence and vice versa. This method therefore allows to conveniently monitor the refolding properties of a protein and to determine its melting temperature, which corresponds to the midpoint of the fluorescence transition curve. The refolding properties and melting temperature of a protein can also be measured by CD spectroscopy, whereby the thermal melting curve of the protein is determined by measuring the CD signal at 222 nm. For purposes of measuring the thermostability, the protein may be dissolved in PBS. For example, the thermostability of a helical protein, such as an ankyrin repeat domain, can be determined by measuring the CD signal of the protein at 222 nm while slowly heating the protein at a concentration of 0.01 mM in PBS pH 7.4 from 20°C to 95°C using a temperature ramp of 1°C per min. A denaturant, such as guanidine chloride, may be added to the PBS buffer, e.g., if measuring a protein that does not fully unfold at 95°C in PBS.

In some embodiments, the increase in melting temperature of the ankyrin repeat domain of the invention is at least 1°C, at least 2°C, at least 3°C, at least 4°C or at least 5°C, as compared to the reference ankyrin repeat domain(s).

In some embodiments, the fraction of the refolded ankyrin repeat domains after thermal denaturation is at least 1%, at least 5%, at least 10% or at least 20% higher, as compared to the reference ankyrin repeat domain(s).

Unless specified, the sequence of the ankyrin repeat domain is not particularly limited. In particular, the ankyrin repeat domain allows for a large sequence variation while preserving the overall structure and function of the domain.

In some embodiments, the N-terminal capping module is derived from the GA-binding protein, e.g, the GA-binding domain having the sequence of chain B of the PDB entry 1AWC. N-terminal capping modules with sequences similar to the N-terminal capping module of the GA-binding protein capping module find reflection in the sequences of SEQ ID NOs: 1 to 37 and the N-terminal capping modules of the ankyrin repeat domains used in the examples. In some embodiments, the N-terminal capping module comprises an amino acid sequence that has at least 70%, at least 75%, at least 80%, at least 85%, at least 90%, at least 95%, or 100% sequence identity to an amino acid sequence selected from the group consisting of SEQ ID NOs: 1 to 38. In some embodiments, the N-terminal capping module comprises an amino acid sequence that has at least 70%, at least 75%, at least 80%, at least 85%, at least 90%, at least 95%, or 100% sequence identity to an amino acid sequence selected from the group consisting of SEQ ID NOs: 1 to 37. In some embodiments, the N-terminal capping module comprises an amino acid sequence that has at least 70%, at least 75%, at least 80%, at least 85%, at least 90%, at least 95%, or 100% sequence identity to an amino acid sequence selected from the group consisting of SEQ ID NOs: 10 to 37. In some embodiments, the N-terminal capping module comprises an amino acid sequence that has at least 70%, at least 75%, at least 80%, at least 85%, at least 90%, at least 95%, or 100% sequence identity to an amino acid sequence selected from the group consisting of SEQ ID NOs: 20 to 37. In some embodiments, the N-terminal capping module comprises an amino acid sequence that has at least 70%, at least 75%, at least 80%, at least 85%, at least 90%, at least 95%, or 100% sequence identity to SEQ ID NO: 35. In some embodiments, the N-terminal capping module comprises an amino acid sequence that has at least 70%, at least 75%, at least 80%, at least 85%, at least 90%, at least 95%, or 100% sequence identity to SEQ ID NO: 38. In some embodiments, the N-terminal capping module comprises an amino acid sequence that has at least 70%, at least 75%, at least 80%, at least 85%, at least 90%, at least 95%, or 100% sequence identity to positions 13 to 42 of an amino acid sequence selected from the group consisting of SEQ ID NOs: 63 to 84, such as positions 13 to 42 of an amino acid sequence selected from the group consisting of SEQ ID NOs: 66 to 68. In some embodiments, the N-terminal capping module comprising any of the amino acid sequences or amino acid sequence variants of this paragraph excludes those variants of the N-terminal capping module comprising the amino acid sequence TPLH.

The N-terminal capping module may further comprise a sequence directly N-terminal to the amino acid sequences defined in SEQ ID NOs: 1 to 38 (or the sequence variants thereof defined herein). For instance, such sequence could be a dipeptide comprising amino acid residues selected from the group consisting of D, A, E, N, Q, S, T, K, R and H, such as the dipeptide GS, DA, EA, AA, AD, AE, NA, AN, PT, TP, AT or TA. For instance, G and S or D and A could be at positions -2 and -1 of the N-terminal capping module, respectively. Such dipeptide sequence may serve as a linker to connect the ankyrin repeat domain to the further peptide sequence of the protein or as an extended alpha-helix of the N-terminal capping module.

In some embodiments, the internal ankyrin repeat(s) of the ankyrin repeat domain consist of 33 amino acid residues.

In some embodiments, the internal ankyrin repeat that is adjacent to the N-terminal capping module comprises an amino acid sequence that has at least 70%, at least 75%, at least 80%, at least 85%, at least 90%, at least 95%, or 100% sequence identity to an amino acid sequence selected from the group consisting of SEQ ID NOs: 39 to 46. In some embodiments, the internal ankyrin repeat that is adjacent to the N-terminal capping module comprises an amino acid sequence that has at least 70%, at least 75%, at least 80%, at least 85%, at least 90%, at least 95%, or 100% sequence identity to an amino acid sequence selected from the group consisting of SEQ ID NOs: 40 to 46. In some embodiments, the internal ankyrin repeat that is adjacent to the N-terminal capping module comprises an amino acid sequence that has at least 70%, at least 75%, at least 80%, at least 85%, at least 90%, at least 95%, or 100% sequence identity to an amino acid sequence of SEQ ID NO: 39. In some embodiments, the internal ankyrin repeat that is adjacent to the N-terminal capping module comprises an amino acid sequence that has at least 70%, at least 75%, at least 80%, at least 85%, at least 90%, at least 95%, or 100% sequence identity to an amino acid sequence of SEQ ID NO: 43. In some embodiments, the internal ankyrin repeat that is adjacent to the N-terminal capping module comprises an amino acid sequence that has at least 70%, at least 75%, at least 80%, at least 85%, at least 90%, at least 95%, or 100% sequence identity to positions 43 to 75 of an amino acid sequence selected from the group consisting of SEQ ID NOs: 63 to 84, such as positions 43 to 75 of an amino acid sequence selected from the group consisting of SEQ ID NOs: 63 to 65.

In some embodiments, each internal ankyrin repeat (of the one or more internal ankyrin repeats) of the ankyrin repeat domain comprises an amino acid sequence as defined above for the internal ankyrin repeat that is adjacent to the N-terminal capping module. Accordingly, if the ankyrin repeat domain has only one internal ankyrin repeat, this internal ankyrin repeat may have an amino acid sequence as defined above. Similarly, if the ankyrin repeat domain has two internal ankyrin repeats, both internal ankyrin repeats may have an amino acid sequence as defined above and so on. In some embodiments, each internal ankyrin repeat comprises an amino acid sequence that has at least 70%, at least 75%, at least 80%, at least 85%, at least 90%, at least 95%, or 100% sequence identity to an amino acid sequence selected from the group consisting of SEQ ID NOs: 39 to 46, such as an amino acid sequence selected from the group consisting of SEQ ID NOs: 40 to 46.

In some embodiments having more than one internal ankyrin repeat, the internal ankyrin repeats share a high degree of sequence identity. In some embodiments, the internal ankyrin repeats share at least 70%, at least 75%, at least 80%, at least 85%, at least 90% or at least 95% sequence identity.

In some embodiments, the C-terminal capping module comprises an amino acid sequence that has at least 70%, at least 75%, at least 80%, at least 85%, at least 90%, at least 95%, or 100% sequence identity to an amino acid sequence selected from the group consisting of SEQ ID NOs: 47 to 59. In some embodiments, the C-terminal capping module comprises an amino acid sequence that has at least 70%, at least 75%, at least 80%, at least 85%, at least 90%, at least 95%, or 100% sequence identity (i) to positions 76 to 103 of an amino acid sequence selected from the group consisting of SEQ ID NOs: 63 to 74 or (ii) to positions 142 to 169 of an amino acid sequence selected from the group consisting of SEQ ID NOs: 75 to 84.

In some embodiments, the N-terminal capping module comprises an amino acid sequence that has at least 70%, at least 75%, at least 80%, at least 85%, at least 90%, at least 95%, or 100% sequence identity to an amino acid sequence selected from the group consisting of SEQ ID NOs: 1 to 37 and the internal ankyrin repeat that is adjacent to the N-terminal capping module comprises an amino acid sequence that has at least 70%, at least 75%, at least 80%, at least 85%, at least 90%, at least 95%, or 100% sequence identity to an amino acid sequence selected from the group consisting of SEQ ID NOs: 40 to 46. In some embodiments, the N-terminal capping module comprises an amino acid sequence that has at least 70% sequence identity to an amino acid sequence selected from the group consisting of SEQ ID NOs: 1 to 37 and the internal ankyrin repeat that is adjacent to the N-terminal capping module comprises an amino acid sequence that has at least 70% sequence identity to an amino acid sequence selected from the group consisting of SEQ ID NOs: 40 to 46. In some embodiments, the N-terminal capping module comprises an amino acid sequence that has at least 75% sequence identity to an amino acid sequence selected from the group consisting of SEQ ID NOs: 1 to 37 and the internal ankyrin repeat that is adjacent to the N-terminal capping module comprises an amino acid sequence that has at least 75% sequence identity to an amino acid sequence selected from the group consisting of SEQ ID NOs: 40 to 46. In some embodiments, the N-terminal capping module comprises an amino acid sequence that has at least 80% sequence identity to an amino acid sequence selected from the group consisting of SEQ ID NOs: 1 to 37 and the internal ankyrin repeat that is adjacent to the N-terminal capping module comprises an amino acid sequence that has at least 80% sequence identity to an amino acid sequence selected from the group consisting of SEQ ID NOs: 40 to 46. In some embodiments, the N-terminal capping module comprises an amino acid sequence that has at least 85% sequence identity to an amino acid sequence selected from the group consisting of SEQ ID NOs: 1 to 37 and the internal ankyrin repeat that is adjacent to the N-terminal capping module comprises an amino acid sequence that has at least 85% sequence identity to an amino acid sequence selected from the group consisting of SEQ ID NOs: 40 to 46. In some embodiments, the N-terminal capping module comprises an amino acid sequence that has at least 90% sequence identity to an amino acid sequence selected from the group consisting of SEQ ID NOs: 1 to 37 and the internal ankyrin repeat that is adjacent to the N-terminal capping module comprises an amino acid sequence that has at least 90% sequence identity to an amino acid sequence selected from the group consisting of SEQ ID NOs: 40 to 46. In some embodiments, the N-terminal capping module comprises an amino acid sequence that has at least 95% sequence identity to an amino acid sequence selected from the group consisting of SEQ ID NOs: 1 to 37 and the internal ankyrin repeat that is adjacent to the N-terminal capping module comprises an amino acid sequence that has at least 95% sequence identity to an amino acid sequence selected from the group consisting of SEQ ID NOs: 40 to 46. In some embodiments, the N-terminal capping module comprises an amino acid sequence that has 100% sequence identity to an amino acid sequence selected from the group consisting of SEQ ID NOs: 1 to 37 and the internal ankyrin repeat that is adjacent to the N-terminal capping module comprises an amino acid sequence that has 100% sequence identity to an amino acid sequence selected from the group consisting of SEQ ID NOs: 40 to 46.

In some embodiments, the N-terminal capping module comprises an amino acid sequence that has at least 70%, at least 75%, at least 80%, at least 85%, at least 90%, at least 95%, or 100% sequence identity to an amino acid sequence selected from the group consisting of SEQ ID NOs: 1 to 37, the internal ankyrin repeat that is adjacent to the N-terminal capping module comprises an amino acid sequence that has at least 70%, at least 75%, at least 80%, at least 85%, at least 90%, at least 95%, or 100% sequence identity to an amino acid sequence selected from the group consisting of SEQ ID NOs: 40 to 46, and the C-terminal capping module comprises an amino acid sequence that has at least 70%, at least 75%, at least 80%, at least 85%, at least 90%, at least 95%, or 100% sequence identity to an amino acid sequence selected from the group consisting of SEQ ID NOs: 47 to 59. In some embodiments, the N-terminal capping module comprises an amino acid sequence that has at least 70%, at least 75%, at least 80%, at least 85%, at least 90%, at least 95%, or 100% sequence identity to an amino acid sequence selected from the group consisting of SEQ ID NOs: 1 to 37, each internal ankyrin repeat comprises an amino acid sequence that has at least 70%, at least 75%, at least 80%, at least 85%, at least 90%, at least 95%, or 100% sequence identity to an amino acid sequence selected from the group consisting of SEQ ID NOs: 40 to 46, and the C-terminal capping module comprises an amino acid sequence that has at least 70%, at least 75%, at least 80%, at least 85%, at least 90%, at least 95%, or 100% sequence identity to an amino acid sequence selected from the group consisting of SEQ ID NOs: 47 to 59. In some embodiments, the sequence identity to the above sequences of the N-terminal capping module, internal ankyrin repeat and C-terminal capping module in this paragraph is at least 70%. In some embodiments, the sequence identity to the above sequences of the N-terminal capping module, internal ankyrin repeat and C-terminal capping module in this paragraph is at least 75%. In some embodiments, the sequence identity to the above sequences of the N-terminal capping module, internal ankyrin repeat and C-terminal capping module in this paragraph is at least 80%. In some embodiments, the sequence identity to the above sequences of the N-terminal capping module, internal ankyrin repeat and C-terminal capping module in this paragraph is at least 85%. In some embodiments, the sequence identity to the above sequences of the N-terminal capping module, internal ankyrin repeat and C-terminal capping module in this paragraph is at least 90%. In some embodiments, the sequence identity to the above sequences of the N-terminal capping module, internal ankyrin repeat and C-terminal capping module in this paragraph is at least 95%. In some embodiments, the sequence identity to the above sequences of the N-terminal capping module, internal ankyrin repeat and C-terminal capping module in this paragraph is 100%.

It is understood that for those embodiments of the N-terminal capping module or internal ankyrin repeat that is adjacent to the N-terminal capping module, which are defined by a certain amino acid residue(s) in a position(s), e.g., L at position 23 of the internal ankyrin repeat that is adjacent to the N-terminal capping module, as well as a minimal sequence identity to an amino acid sequence, both conditions need to be fulfilled. For instance, an internal ankyrin repeat that is adjacent to the N-terminal capping module having L at position 23 and at least 70% sequence identity to an amino acid sequence selected from the group consisting of SEQ ID NOs: 40 to 46, only relates to such embodiments wherein the internal ankyrin repeat that is adjacent to the N-terminal capping module has L at position 23 and, at the same time, at least 70% sequence identity to one or more of SEQ ID NOs: 40 to 46.

In some embodiments, the protein of the invention comprises an ankyrin repeat domain, wherein the internal ankyrin repeat that is adjacent to the N-terminal capping module (a) has an amino acid residue of the leucine class selected from L and I at position 23 and (b) comprises an amino acid sequence that has at least 70% sequence identity to an amino acid sequence selected from the group consisting of SEQ ID NOs: 40 to 46, and wherein the N-terminal capping module (A) has an amino acid residue selected from the group consisting of I, T, A, V, L and M at position 15 and/or an amino acid residue selected from the group consisting of L, V, I and A at position 22 and (B) comprises an amino acid sequence that has at least 70% sequence identity to an amino acid sequence selected from the group consisting of SEQ ID NOs: 1 to 37, and, optionally, wherein the ankyrin repeat domain has an improved thermostability, such as a higher melting temperature and/or a higher fraction of refolded ankyrin repeat domains after thermal denaturation, as compared to a reference ankyrin repeat domain having the same amino acid sequence except for one or more of these mutations. In some embodiments, the protein of the invention comprises an ankyrin repeat domain, wherein the internal ankyrin repeat that is adjacent to the N-terminal capping module (a) has an amino acid residue of the leucine class selected from L and I at position 23 and (b) comprises an amino acid sequence that has at least 70% sequence identity to an amino acid sequence selected from the group consisting of SEQ ID NOs: 40 to 46, and wherein the N-terminal capping module (A) has an amino acid residue selected from the group consisting of I, T, A, V, L and M at position 15 and (B) comprises an amino acid sequence that has at least 70% sequence identity to an amino acid sequence selected from the group consisting of SEQ ID NOs: 1 to 37, and, optionally, wherein the ankyrin repeat domain has an improved thermostability, such as a higher melting temperature and/or a higher fraction of refolded ankyrin repeat domains after thermal denaturation, as compared to a first reference ankyrin repeat domain having the same amino acid sequence except for the mutation at position 23 of the internal ankyrin repeat that is adjacent to the N-terminal capping module, which is V in the first reference ankyrin repeat domain, and/or as compared to a second reference ankyrin repeat domain having the same amino acid sequence except for position 15 of the N-terminal capping module, which is D in the second reference ankyrin repeat domain. In some embodiments, the protein of the invention comprises an ankyrin repeat domain, wherein the internal ankyrin repeat that is adjacent to the N-terminal capping module (a) has an amino acid residue of the leucine class selected from L and I at position 23 and (b) comprises an amino acid sequence that has at least 70% sequence identity to an amino acid sequence selected from the group consisting of SEQ ID NOs: 40 to 46, and wherein the N-terminal capping module (A) has an amino acid residue selected from the group consisting of I, V and L at position 15 and (B) comprises an amino acid sequence that has at least 70% sequence identity to an amino acid sequence selected from the group consisting of SEQ ID NOs: 1 to 37, and, optionally, wherein the ankyrin repeat domain has an improved thermostability, such as a higher melting temperature and/or a higher fraction of refolded ankyrin repeat domains after thermal denaturation, as compared to a first reference ankyrin repeat domain having the same amino acid sequence except for the mutation at position 23 of the internal ankyrin repeat that is adjacent to the N-terminal capping module, which is V in the first reference ankyrin repeat domain, and/or as compared to a second reference ankyrin repeat domain having the same amino acid sequence except for position 15 of the N-terminal capping module, which is D in the second reference ankyrin repeat domain. In some embodiments, the protein of the invention comprises an ankyrin repeat domain, wherein the internal ankyrin repeat that is adjacent to the N-terminal capping module (a) has an amino acid residue of the leucine class selected from L and I at position 23 and (b) comprises an amino acid sequence that has at least 70% sequence identity to an amino acid sequence selected from the group consisting of SEQ ID NOs: 40 to 46, and wherein the N-terminal capping module (A) has I at position 15 and (B) comprises an amino acid sequence that has at least 70% sequence identity to an amino acid sequence selected from the group consisting of SEQ ID NOs: 1 to 37, and, optionally, wherein the ankyrin repeat domain has an improved thermostability, such as a higher melting temperature and/or a higher fraction of refolded ankyrin repeat domains after thermal denaturation, as compared to a first reference ankyrin repeat domain having the same amino acid sequence except for the mutation at position 23 of the internal ankyrin repeat that is adjacent to the N-terminal capping module, which is V in the first reference ankyrin repeat domain, and/or as compared to a second reference ankyrin repeat domain having the same amino acid sequence except for position 15 of the N-terminal capping module, which is D in the second reference ankyrin repeat domain. In some embodiments, the protein of the invention comprises an ankyrin repeat domain, wherein the internal ankyrin repeat that is adjacent to the N-terminal capping module (a) has L at position 23 and (b) comprises an amino acid sequence that has at least 70% sequence identity to an amino acid sequence selected from the group consisting of SEQ ID NOs: 40 to 46, and wherein the N-terminal capping module (A) has I at position 15 and (B) comprises an amino acid sequence that has at least 70% sequence identity to an amino acid sequence selected from the group consisting of SEQ ID NOs: 1 to 37, and, optionally, wherein the ankyrin repeat domain has an improved thermostability, such as a higher melting temperature and/or a higher fraction of refolded ankyrin repeat domains after thermal denaturation, as compared to a first reference ankyrin repeat domain having the same amino acid sequence except for the mutation at position 23 of the internal ankyrin repeat that is adjacent to the N-terminal capping module, which is V in the first reference ankyrin repeat domain, and/or as compared to a second reference ankyrin repeat domain having the same amino acid sequence except for position 15 of the N-terminal capping module, which is D in the second reference ankyrin repeat domain. In some embodiments, the protein of the invention comprises an ankyrin repeat domain, wherein the internal ankyrin repeat that is adjacent to the N-terminal capping module (a) has I at position 23 and (b) comprises an amino acid sequence that has at least 70% sequence identity to an amino acid sequence selected from the group consisting of SEQ ID NOs: 40 to 46, and wherein the N-terminal capping module (A) has I at position 15 and (B) comprises an amino acid sequence that has at least 70% sequence identity to an amino acid sequence selected from the group consisting of SEQ ID NOs: 1 to 37, and, optionally, wherein the ankyrin repeat domain has an improved thermostability, such as a higher melting temperature and/or a higher fraction of refolded ankyrin repeat domains after thermal denaturation, as compared to a first reference ankyrin repeat domain having the same amino acid sequence except for the mutation at position 23 of the internal ankyrin repeat that is adjacent to the N-terminal capping module, which is V in the first reference ankyrin repeat domain, and/or as compared to a second reference ankyrin repeat domain having the same amino acid sequence except for position 15 of the N-terminal capping module, which is D in the second reference ankyrin repeat domain. In some embodiments, the protein of the invention comprises an ankyrin repeat domain, wherein the internal ankyrin repeat that is adjacent to the N-terminal capping module (a) has an amino acid residue of the leucine class selected from L and I at position 23 and (b) comprises an amino acid sequence that has at least 70% sequence identity to an amino acid sequence selected from the group consisting of SEQ ID NOs: 40 to 46, and wherein the N-terminal capping module (A) has an amino acid residue selected from the group consisting of L, V and I at position 22 and (B) comprises an amino acid sequence that has at least 70% sequence identity to an amino acid sequence selected from the group consisting of SEQ ID NOs: 1 to 37, and, optionally, wherein the ankyrin repeat domain has an improved thermostability, such as a higher melting temperature and/or a higher fraction of refolded ankyrin repeat domains after thermal denaturation, as compared to a first reference ankyrin repeat domain having the same amino acid sequence except for the mutation at position 23 of the internal ankyrin repeat that is adjacent to the N-terminal capping module, which is V in the first reference ankyrin repeat domain, and/or as compared to a second reference ankyrin repeat domain having the same amino acid sequence except for position 22 of the N-terminal capping module, which is M in the second reference ankyrin repeat domain. In some embodiments, the protein of the invention comprises an ankyrin repeat domain, wherein the internal ankyrin repeat that is adjacent to the N-terminal capping module (a) has an amino acid residue of the leucine class selected from L and I at position 23 and (b) comprises an amino acid sequence that has at least 70% sequence identity to an amino acid sequence selected from the group consisting of SEQ ID NOs: 40 to 46, and wherein the N-terminal capping module (A) has L at position 24 and (B) comprises an amino acid sequence that has at least 70% sequence identity to an amino acid sequence selected from the group consisting of SEQ ID NOs: 1 to 37, and, optionally, wherein the ankyrin repeat domain has an improved thermostability, such as a higher melting temperature and/or a higher fraction of refolded ankyrin repeat domains after thermal denaturation, as compared to a first reference ankyrin repeat domain having the same amino acid sequence except for the mutation at position 23 of the internal ankyrin repeat that is adjacent to the N-terminal capping module, which is V in the first reference ankyrin repeat domain, and/or as compared to a second reference ankyrin repeat domain having the same amino acid sequence except for position 24 of the N-terminal capping module, which is A in the second reference ankyrin repeat domain. In some embodiments, the protein of the invention comprises an ankyrin repeat domain, wherein the internal ankyrin repeat that is adjacent to the N-terminal capping module (a) has an amino acid residue of the leucine class selected from L and I at position 23 and (b) comprises an amino acid sequence that has at least 70% sequence identity to an amino acid sequence selected from the group consisting of SEQ ID NOs: 40 to 46, and wherein the N-terminal capping module (A) has the amino acid sequence KELIAL at positions 19 to 24 and (B) comprises an amino acid sequence that has at least 70% sequence identity to an amino acid sequence selected from the group consisting of SEQ ID NOs: 1 to 37, and, optionally, wherein the ankyrin repeat domain has an improved thermostability, such as a higher melting temperature and/or a higher fraction of refolded ankyrin repeat domains after thermal denaturation, as compared to a reference ankyrin repeat domain having the same amino acid sequence except for the mutation at position 23 of the internal ankyrin repeat that is adjacent to the N-terminal capping module, which is V in the reference ankyrin repeat domain. In some embodiments, the internal ankyrin repeat that is adjacent to the N-terminal capping module and the N-terminal capping module of the above embodiments in this paragraph each have at least 75% sequence identity to the indicated sequences. In some embodiments, the internal ankyrin repeat that is adjacent to the N-terminal capping module and the N-terminal capping module of the above embodiments in this paragraph each have at least 80% sequence identity to the indicated sequences. In some embodiments, the internal ankyrin repeat that is adjacent to the N-terminal capping module and the N-terminal capping module of the above embodiments in this paragraph each have at least 85% sequence identity to the indicated sequences. In some embodiments, the internal ankyrin repeat that is adjacent to the N-terminal capping module and the N-terminal capping module of the above embodiments in this paragraph each have at least 90% sequence identity to the indicated sequences. In some embodiments, the internal ankyrin repeat that is adjacent to the N-terminal capping module and the N-terminal capping module of the above embodiments in this paragraph each have at least 95% sequence identity to the indicated sequences. In some embodiments, the internal ankyrin repeat that is adjacent to the N-terminal capping module and the N-terminal capping module of the above embodiments in this paragraph each have 100% sequence identity to the indicated sequences.

In some embodiments, the ankyrin repeat domain comprises the N-terminal capping module, one internal ankyrin repeat and a C-terminal capping module (such ankyrin repeat domain structure is also referred to as "N1C"). Such ankyrin repeat domains are shown in Example 1. In some embodiments, the ankyrin repeat domain comprises the N-terminal capping module, multiple internal ankyrin repeats, such as 2, 3, 4 or 5 internal ankyrin repeats, and a C-terminal capping module. For instance, the ankyrin repeat domain may comprise the N-terminal capping module, multiple internal ankyrin repeats comprising the sequence of SEQ ID NO: 46, such as 2, 3, 4 or 5 of such internal ankyrin repeats, and a C-terminal capping module. In one embodiment, the ankyrin repeat domain comprises the N-terminal capping module, 2 or 3 internal ankyrin repeats and a C-terminal capping module (such ankyrin repeat domain structure is also referred to as "N2C" or "N3C", respectively). In one embodiment, the ankyrin repeat domain has a N2C structure. In another embodiment, the ankyrin repeat domain has a N3C structure.

In some embodiments, the protein of the invention is a recombinant protein or a DARPin.

In some embodiments, the ankyrin repeat domain of the protein of the invention specifically binds to a target. For instance, the ankyrin repeat domain may specifically bind to a mammalian serum albumin, such as human serum albumin. Exemplary ankyrin repeat domains specifically binding to human serum albumin are disclosed in WO 2012/069654 A1 and also found in ensovibep (see amino acid residues 1-126 and 149-274 of ensovibep, respectively, as defined, e.g., in Proposed INN List: 124; WHO Drug Information, Vol. 34, No. 4, 2020). In some embodiments, the target is a peptide-MHC complex. In some embodiments, the target is a protein on a cell surface, such as Her2, CD3, CD4, CD8, CD33, CD40, CD70, CD123, FAP or 4-1BB. In some embodiments, the target is an intracellular protein. In some embodiments, the target is a protein on the surface of a virus, such as the spike protein of SARS-CoV-2. In some embodiments, the target is a blood-circulating protein, such as VEGF.

In some embodiments, the protein only comprises a single ankyrin repeat domain.

The protein may also comprise one or more further moieties in addition to the ankyrin repeat domain having the internal ankyrin repeat that is adjacent to the N-terminal capping module with the amino acid residue of the leucine class at position 23, such as a moiety binding to a target, a labeling moiety, a toxic moiety, a moiety improving the pharmacokinetics, a moiety providing effector functions, a moiety allowing for the purification of the protein or a moiety providing enzymatic activity. In some embodiments, the further moiety binding to a target is another ankyrin repeat domain, an antibody or fragment thereof or a receptor protein. In some embodiments, the further moiety binding to a target is another ankyrin repeat domain. In some embodiments, the labeling moiety is a stable isotope, a mass tag or a fluorescent label. In some embodiments, the toxic moiety is a chemotherapeutic agent, such as an alkylating agent, an antimetabolite, a taxane, or an anthracycline. In some embodiments, the moiety improving pharmacokinetics is a polypeptide (e.g., as used for PASylation), polyethylene glycol (PEG), a mammalian serum albumin, an immunoglobulin, a Fc domain of an immunoglobulin or a moiety binding to mammalian serum albumin or to an immunoglobulin. In one embodiment, the protein further contains an ankyrin repeat domain binding to a mammalian serum albumin. In some embodiments, the further moiety providing effector functions is a Fc domain of an immunoglobulin. In some embodiments, the moiety allowing for the purification of the protein is a FLAG-tag, a GST-tag, an HA-tag, a Myc-tag, a His-tag or a Strep-tag. In some embodiments, the further moiety providing enzymatic or fluorescence activity is, e.g., beta-lactamase or green fluorescence protein, respectively.

The further moiety may be proteinaceous or non-proteinaceous.

In some embodiments, the further moiety in addition to the ankyrin repeat domain having the internal ankyrin repeat that is adjacent to the N-terminal capping module with the amino acid residue of the leucine class at position 23 is one or more additional ankyrin repeat domain(s). In some embodiments, one or more of the additional ankyrin repeat domain(s) is an ankyrin repeat domain of the invention and thus also has an amino acid residue of the leucine class, such as L or I, at position 23 of the internal ankyrin repeat that is adjacent to the N-terminal capping module. In some embodiments, none of the additional one or more ankyrin repeat domain(s) has an amino acid residue of the leucine class, such as L or I, at position 23 of the internal ankyrin repeat that is adjacent to the N-terminal capping module. In some embodiments, all of the additional ankyrin repeat domain(s) are ankyrin repeat domains of the invention. In some embodiments, the protein of the invention comprises more than one, e.g., at least two, at least three, at least four, at least five, or at least six, ankyrin repeat domains having an amino acid residue of the leucine class, such as L or I, at position 23 of the internal ankyrin repeat that is adjacent to the N-terminal capping module and the same one or more mutation(s) in the N-terminal capping module, for instance, an amino acid residue selected from the group consisting of I, T, A, V, L and M at position 15 of the N-terminal capping module. In some embodiments, the protein of the invention comprises more than one, e.g., at least two, at least three, at least four, at least five, or at least six, ankyrin repeat domains. In some embodiments, the protein of the invention comprises more than one, e.g., at least two, at least three, at least four, at least five, or at least six, ankyrin repeat domains each corresponding to an ankyrin repeat domain of the invention. In some embodiments, the protein of the invention comprises only one ankyrin repeat domain.

In some embodiments, the protein of the invention is multivalent, i.e. it comprises multiple identical moieties binding to the same target, in particular multiple identical ankyrin repeat domains binding to the same target. In some embodiments, the protein is bivalent, trivalent, tetravalent, pentavalent or hexavalent. In some embodiments, the protein of the invention is multiparatopic, i.e. it comprises multiple different moieties binding to the same target, in particular multiple different ankyrin repeat domains binding to the same target. In some embodiments, the protein is biparatopic, triparatopic, tetraparatopic, pentaparatopic or hexaparatopic. In some embodiments, the protein of the invention is multispecific, i.e. it comprises multiple different moieties binding to different targets, in particular multiple different ankyrin repeat domains binding to different targets. In some embodiments, the protein is bispecific, trispecific, tetraspecific, pentaspecific or hexaspecific. In some embodiments, the multivalent, multiparatopic or multispecific protein has more than one ankyrin repeat domain of the invention. In some embodiments, the multivalent, multiparatopic or multispecific protein has ankyrin repeat domains that are all ankyrin repeat domains of the invention.

The various moieties of the protein, including said ankyrin repeat domain of the invention, may connect covalently and/or non-covalently to one another. The various moieties may connect covalently to one another, for instance, via a peptide linker or via a maleimidecontaining crosslinker. Suitable peptide linkers include glycine-serine linkers and proline-threonine linkers. In some embodiments, the suitable peptide linker is a naturally found peptide linker, such as the IgG hinge region. In some embodiments, the peptide linkers have a length of 2 to 24 amino acid residues or 2 to 16 amino acid residues. Exemplary peptide linkers include the linkers of SEQ ID NOs: 60 to 62. The various moieties may also connect non-covalently to one another, for instance, via a multimerization moiety. In some embodiments, a multimerization moiety is an immunoglobulin heavy chain constant region, a leucine zipper or a free thiol which can form a disulfide bond with another free thiol.

In some embodiments, the protein comprises one or more additional ankyrin repeat domains as further moieties that are connected by a proline-threonine linker.

The ankyrin repeat domain of the invention may be derived from various methods, such as selection from a protein library, *in silico* design or by mutating an existing ankyrin repeat domain. Subsequently, the protein comprising the ankyrin repeat domain of the invention (and possibly one or more further moieties) may be expressed or synthesized by methods known in the art and, e.g., formulated as a pharmaceutical product. Accordingly, in a further aspect, the present disclosure relates to a library of proteins comprising one or more proteins of the invention. In some embodiments, the protein library comprises at least 10³, at least 10⁵, at least 10⁷, at least 10⁹, at least 10¹⁰, at least 10¹¹, at least 10¹² or at least 10¹³ proteins, each protein comprising an ankyrin repeat domain, and the library comprising one or more proteins of the invention. In some embodiments, the protein library comprises at least 10³, at least 10⁵, at least 10⁷, at least 10⁹, at least 10¹⁰, at least 10¹¹, at least 10¹² or at least 10¹³ proteins of the invention. In some embodiments, the protein library comprises at least 10³, at least 10⁵, at least 10⁷, at least 10⁹, at least 10¹⁰, at least 10¹¹, at least 10¹² or at least 10¹³ proteins that differ in the amino acid sequence of their ankyrin repeat domain and the library comprising one or more proteins of the invention. In some embodiments, the protein library comprises at least 10³, at least 10⁵, at least 10⁷, at least 10⁹, at least 10¹⁰, at least 10¹¹, at least 10¹² or at least 10¹³ proteins of the invention that differ in the amino acid sequence of their ankyrin repeat domain. In some embodiments, substantially all proteins of the protein library differ in the amino acid sequence of their ankyrin repeat domain. In some embodiments, the protein library exclusively comprises proteins of the invention. In some embodiments, the protein library comprises at least one protein of the invention.

In some embodiments, the protein library comprises proteins having ankyrin repeat domains with different structures. For instance, the protein library may contain a mixture of proteins comprising N2C and N3C ankyrin repeat domains. In some embodiments, the structure of the ankyrin repeat domain is identical for all proteins of the library, e.g., the ankyrin repeat domain of all proteins is either exclusively of N2C structure or exclusively of N3C structure. In some embodiments, the ankyrin repeat domain of all proteins is of the N2C structure. In other embodiments, the ankyrin repeat domain of all proteins is of the N3C structure. In some embodiments, the proteins of the library each comprise a single ankyrin repeat domain only.

The sequence variability in the ankyrin repeat domains of the protein library may be brought about randomly, e.g., by error-prone PCR of the nucleic acid molecules encoding the proteins, or it may be obtained by rational design followed by, e.g., direct synthesis of the nucleic acid molecules encoding the proteins ("design approach"). In some embodiments, the variability is introduced by the design approach. In the design approach, variability of the amino acid sequence is introduced in one or more than one position of the ankyrin repeat domains. The variable positions that may be occupied by different amino acid residues are also referred to as "randomized positions", whereas the positions that are always occupied by the same amino acid residue are referred to as "fixed positions". In some embodiments, the randomized positions are those positions occupied by potential target interaction residues and/or the fixed positions are those positions occupied by framework residues. In some embodiments, one or more of the positions occupied by potential target interaction residues are randomized positions. In some embodiments, all positions occupied by potential target interaction residues are randomized positions. In some embodiments, one or more of the positions occupied by framework residues are fixed positions. In some embodiments, all positions occupied by framework residues are fixed positions.

In certain embodiments, there are corresponding fixed positions and randomized positions in the ankyrin repeat domain of the different proteins of the protein library. Due to the intended variability in the randomized positions, the amino acid residues in corresponding randomized position may differ, although there may also be identical amino acid residues in corresponding randomized positions for at least some of the proteins in the library (though, in such cases, the proteins will not necessarily have identical amino acid residues in each of their corresponding randomized positions). In some embodiments, the fixed positions and the randomized positions are the same for the ankyrin repeat domains of each protein of the protein library. In some embodiments of ankyrin repeat domains having multiple internal ankyrin repeats, the internal ankyrin repeats of each ankyrin repeat domain have different randomized and fixed positions. In some embodiments of ankyrin repeat domains having multiple internal ankyrin repeats, the internal ankyrin repeats of each ankyrin repeat domain have different randomized and fixed positions and the fixed positions and the randomized positions are the same for the ankyrin repeat domains of each protein of the protein library. In some embodiments of ankyrin repeat domains having multiple internal ankyrin repeats, the internal ankyrin repeats of each ankyrin repeat domain have the same randomized and fixed positions. In some embodiments of ankyrin repeat domains having multiple internal ankyrin repeats, the internal ankyrin repeats of each ankyrin repeat domain have the same randomized and fixed positions and the fixed positions and the randomized positions are the same for the ankyrin repeat domains of each protein of the protein library.

The randomized positions may show different degrees of variability, i.e. they may be occupied by different sets of amino acid residues. The "X" amino acid residues of SEQ ID NOs: 40 to 46 and 56 are such randomized positions and may each be occupied by any amino acid residue, as is reflected in the sequence listing. In some embodiments, the degree of variability differs between randomized positions. In some embodiments, the amino acid residue in a randomized position is any of the naturally occurring amino acid residues. In some embodiments, the amino acid residue in all randomized positions is any of the naturally occurring amino acid residues. In some embodiments, one or more randomized position(s) are only occupied by a subset of the naturally occurring amino acid residues. Such subsets can be those having common physicochemical properties, such as sets of hydrophobic, hydrophilic, acidic, basic, aromatic, or aliphatic amino acid residues. Other subsets are those comprising all naturally occurring amino acid residues except for certain non-desired amino acid residues, such as sets not comprising C or P. In some embodiments, one or more randomized position(s) are only occupied by any naturally occurring amino acid residue other than (i) an amino acid residue selected from the group consisting of C, G, M and N if followed by a G amino acid residue and (ii) P. In some embodiments, one or more randomized position(s) are only occupied by any naturally occurring amino acid residue other than C or other than C, G and P. In yet other embodiments, the subsets comprise those amino acid residues that are found in the corresponding positions of naturally occurring ankyrin repeats.

In some embodiments, the proteins of the protein library share at least 70%, at least 75%, at least 80%, at least 85%, at least 90% or at least 95% sequence identity in the amino acid sequence of their ankyrin repeat domains.

The above protein library can serve to select those proteins of the library that have a predetermined property, i.e. a certain property of interest that may be found in the ankyrin repeat domain of one of the proteins of the protein library and that can be screened for. Such predetermined property may include the specific binding to a target, the activation or inhibition of a target, such as an enzyme, and the blocking of an interaction between two targets. In some embodiments, the predetermined property is the specific binding to a target. Preferably, the protein selected from the library is a protein of the invention.

In one embodiment, the present disclosure provides a method for selecting a protein that specifically binds to a target, comprising the following steps:
a) providing a library of proteins comprising one or more proteins of the invention; and
b) selecting a protein specifically binding to the target via its ankyrin repeat domain from the library,
wherein the selected protein is a protein of the invention.

In one embodiment, the present disclosure provides a method for selecting a protein that specifically binds to a target, comprising the following steps:
a) providing a library of proteins of the invention; and
b) selecting a protein specifically binding to the target via its ankyrin repeat domain from the library.

During the selection step b), the proteins can be selected using screening methods commonly known to the person skilled in the art, such as yeast display, protein fragment complementation assay, phage display or ribosome display. The protein may also be selected during selection step b) by screening the library of step a) *in silico.* In some embodiments, the proteins are selected in step b) using phage display or ribosome display.

As indicated above, the protein of the invention as found in the protein library or represented by the protein selected from the library is not particularly limited in the sequence of the ankyrin repeat domain outside position 23 of the internal ankyrin repeat that is adjacent to the N-terminal capping module. In some embodiments, the internal ankyrin repeat that is adjacent to the N-terminal capping module of such ankyrin repeat domain comprises an amino acid sequence that has at least 70%, at least 75%, at least 80%, at least 85%, at least 90%, at least 95%, or 100% sequence identity to an amino acid sequence selected from the group consisting of SEQ ID NOs: 40 to 46. In some embodiments, the thermostability of such ankyrin repeat domain is improved in comparison to a reference ankyrin repeat domain having the same amino acid sequence except for the amino acid residue at position 23 of the internal ankyrin repeat that is adjacent to the N-terminal capping module, which is, e.g., V in the reference ankyrin repeat domain.

After the selection of a protein, the protein can be further modified, mutated and/or optimized by methods commonly known in the art.

For instance, amino acid sequence variants of the protein can be generated, e.g., by subjecting the nucleic acid encoding the selected protein to physical or chemical mutagens, copying said nucleic acid by error-prone PCR, using said nucleic acid for DNA shuffling or random chimeragenesis (Neylon C., Nucleic Acids Res., 32(4), 1448-1459, 2004). The protein library of such amino acid sequence variants may then again be subjected to the above selection step b) in order to select the variant(s) having the predetermined property.

The protein selected in step b) above may also be selectively mutated. For instance, one or more cysteine residues may be introduced, the thiol group(s) of which can then react with maleimide cross-linkers. Similarly, certain non-desirable amino acid residues may be removed, for instance, cysteines, which are prone to oxidations. Also, amino acid residues may be selectively mutated after analysis of the crystal structure so that the protein structure better fits to the target.

The protein selected in step b) may also become modified with one or more further moieties as outlined above for the protein of the invention. In one embodiment, the protein selected in step b) is modified with one or more further ankyrin repeat domains.

In one embodiment, the present disclosure provides a method of modifying a protein comprising an ankyrin repeat domain that does not have one or more mutations specified herein, e.g., one that does not have L or I at position 23 of the internal ankyrin repeat that is adjacent to the N-terminal capping module, by replacing one or more amino acid residues to result in a protein of the invention. By modifying an ankyrin repeat domain in this way, the favorable properties of the ankyrin repeat domain of the invention disclosed herein may be transferred to the ankyrin repeat domain of the thus obtained protein. In some embodiments, the amino acid residue at position 23 of the internal ankyrin repeat that is adjacent to the N-terminal capping module is replaced alone. In other embodiments, the amino acid residue at position 23 of the internal ankyrin repeat that is adjacent to the N-terminal capping module is replaced together with other amino acid residues, e.g., other amino acid residues of the N-terminal capping module as disclosed herein. In some embodiments, one or more of the mutations in the N-terminal capping module referred to above are introduced by replacing the amino acid residue(s) at the corresponding position(s). In some embodiments, the entire N-terminal capping module may be replaced. In some embodiments, the amino acid residue at position 23 of the internal ankyrin repeat that is adjacent to the N-terminal capping module is replaced by L or I and/or the amino acid residue at position 15 of the N-terminal capping module is replaced by I or V. In some embodiments, the amino acid residue at position 23 of the internal ankyrin repeat that is adjacent to the N-terminal capping module is replaced by L and/or the amino acid residue at position 15 of the N-terminal capping module is replaced by I.

Thus, in one embodiment, the present disclosure provides a method of preparing a protein or a method of improving the thermostability of an ankyrin repeat domain comprising the following steps:
a) selecting a protein comprising an ankyrin repeat domain with an internal ankyrin repeat that is adjacent to the N-terminal capping module that does not have L or I at position 23; and
b) replacing one or more amino acid residues of the protein to result in a protein of the invention.

In one embodiment, the present disclosure provides a method of preparing a protein or a method of improving the thermostability of an ankyrin repeat domain comprising the following steps:
a) selecting a protein comprising an ankyrin repeat domain with (1) an internal ankyrin repeat that is adjacent to the N-terminal capping module that does not have L or I at position 23 and/or (2) an N-terminal capping module that does not have an amino acid residue selected from I, T, A, V, L and M at position 15; and
b) replacing one or more amino acid residues of the protein to result in a protein of the invention comprising an ankyrin repeat domain having L or I at position 23 of the internal ankyrin repeat that is adjacent to the N-terminal capping module and having an amino acid residue selected from I, T, A, V, L and M at position 15 of the N-terminal capping module.

In one embodiment, the present disclosure provides a method of preparing a protein or a method of improving the thermostability of an ankyrin repeat domain comprising the following steps:
a) selecting a protein comprising an ankyrin repeat domain with (1) an internal ankyrin repeat that is adjacent to the N-terminal capping module that does not have L or I at position 23 and/or (2) an N-terminal capping module that does not have I at position 15; and
b) replacing one or more amino acid residues of the protein to result in a protein of the invention comprising an ankyrin repeat domain having L or I at position 23 of the internal ankyrin repeat that is adjacent to the N-terminal capping module and having I at position 15 of the N-terminal capping module.

In one embodiment, the present disclosure provides a method of preparing a protein or a method of improving the thermostability of an ankyrin repeat domain comprising the following steps:
a) selecting a protein comprising an ankyrin repeat domain with (1) an internal ankyrin repeat that is adjacent to the N-terminal capping module that does not have L or I at position 23 and/or (2) an N-terminal capping module that does not have an amino acid residue selected from I, L and V at position 22; and
b) replacing one or more amino acid residues of the protein to result in a protein of the invention comprising an ankyrin repeat domain having L or I at position 23 of the internal ankyrin repeat that is adjacent to the N-terminal capping module and having an amino acid residue selected from I, L and V at position 22 of the N-terminal capping module.

In one embodiment, the present disclosure provides a method of preparing a protein or a method of improving the thermostability of an ankyrin repeat domain comprising the following steps:
a) selecting a protein comprising an ankyrin repeat domain with (1) an internal ankyrin repeat that is adjacent to the N-terminal capping module that does not have L or I at position 23 and/or (2) an N-terminal capping module that does not have L at position 24; and
b) replacing one or more amino acid residues of the protein to result in a protein of the invention comprising an ankyrin repeat domain having L or I at position 23 of the internal ankyrin repeat that is adjacent to the N-terminal capping module and having L at position 24 of the N-terminal capping module.

As indicated above, a protein of the invention resulting from the replacement method is not particularly limited in the sequence of the ankyrin repeat domain outside position 23 of the internal ankyrin repeat that is adjacent to the N-terminal capping module. In some embodiments, the internal ankyrin repeat that is adjacent to the N-terminal capping module of the ankyrin repeat domain resulting from the replacement method comprises an amino acid sequence that has at least 70%, at least 75%, at least 80%, at least 85%, at least 90%, at least 95%, or 100% sequence identity to an amino acid sequence selected from the group consisting of SEQ ID NOs: 40 to 46. In some embodiments, the thermostability of the ankyrin repeat domain resulting from the replacement method is improved in comparison to a reference ankyrin repeat domain having the same amino acid sequence except for the amino acid residue at position 23 of the internal ankyrin repeat that is adjacent to the N-terminal capping module, which is, e.g., V in the reference ankyrin repeat domain. In some embodiments, the thermostability of the ankyrin repeat domain of the protein resulting from the replacement method is improved in comparison to the ankyrin repeat domain of the original protein.

The protein resulting from the replacement method can be further modified, mutated and/or optimized by methods commonly known in the art. In some embodiments, the protein resulting from the replacement method comprises one or more further moieties in addition to the ankyrin repeat domain as outlined above for the protein of the invention. Such modification with one or more further moieties may occur before, during or after the replacement of the one or more amino acid residues. In some embodiments, the one or more further moieties are added to the protein after replacement of the one or more amino acid residues. In some embodiments, the one or more further moieties are added to the protein before replacement of the one or more amino acid residues.

The present disclosure also relates to a method of designing or optimizing the amino acid sequence of the ankyrin repeat domain of the protein of the invention *in silico* through computational methods. It is to be understood that the ankyrin repeat domain may be entirely designed *in silico* or partially, e.g., by optimizing a pre-existing ankyrin repeat domain through computational methods.

Thus, in one embodiment, the present disclosure provides a method of designing a protein comprising designing or optimizing the amino acid sequence of an ankyrin repeat domain *in silico* through computational methods to result in a protein of the invention.

As indicated above, a protein of the invention resulting from such design method is not particularly limited in the sequence of said ankyrin repeat domain outside position 23 of the internal ankyrin repeat that is adjacent to the N-terminal capping module. In some embodiments, the internal ankyrin repeat that is adjacent to the N-terminal capping module of the *in silico* designed or optimized ankyrin repeat domain comprises an amino acid sequence that has at least 70%, at least 75%, at least 80%, at least 85%, at least 90%, at least 95%, or 100% sequence identity to an amino acid sequence selected from the group consisting of SEQ ID NOs: 40 to 46. In some embodiments, the thermostability of the designed or optimized ankyrin repeat domain is improved in comparison to a reference ankyrin repeat domain having the same amino acid sequence except for the amino acid residue at position 23 of the internal ankyrin repeat that is adjacent to the N-terminal capping module, which is, e.g., V in the reference ankyrin repeat domain.

The protein comprising the designed or optimized ankyrin repeat domain can be further modified, mutated and/or optimized by methods commonly known in the art. In some embodiments, the protein comprising the designed or optimized ankyrin repeat domain comprises one or more further moieties in addition to the ankyrin repeat domain as outlined above for the protein of the invention. Such modification with one or more further moieties may occur before, during or after the *in silico* design or optimization of the ankyrin repeat domain.

In some embodiments, the protein of the invention, e.g., a protein resulting from one of the above methods, is expressed or synthesized. In some embodiments, the expressed or synthesized protein is purified after its expression or synthesis. In some embodiments, the expressed or synthesized and, optionally, purified protein is formulated as a pharmaceutical composition.

In further aspects, the present disclosure provides a nucleic acid encoding the protein of the invention, a chromosome or vector comprising such nucleic acid, such as a bacterial vector, a viral vector or a synthetic vector (e.g., a lipid nanoparticle (LNP) vector), and a cell or *in vitro* expression system comprising such nucleic acid, chromosome or vector.

The nucleic acid can be DNA or RNA, single-stranded or double-stranded, in isolated form or part of a larger nucleic acid, e.g., of a vector or a chromosome. The nucleic acid may comprise elements that enable delivery of the nucleic acid to a cell and/or expression of the nucleic acid in a cell. For instance, the nucleic acid encoding the protein of the invention can be operatively linked to expression control sequences, which have an impact on the transcription and/or translation of the protein, such as promoters, enhancers, transcription terminators, start codons and stop codons. Depending on the intended application and/or context, the expression control sequences may be selected from any eukaryotic or prokaryotic organism. Suitable promoters may be constitutive or inducible promoters. Examples include the CMV-, lacZ-, T7-, T5-, RSV-, SV40-, AOX1-, and GAPDH-promoter. Suitable enhancers include the CMV-enhancer, insulin-responsive elements, and SV40-enhancer. Suitable transcription terminators include the SV40-, lacZ-, and tk-polyadenylation signal.

The present disclosure also provides a library of nucleic acids comprising one or more nucleic acids encoding a protein of the invention. In some embodiments, the nucleic acid library comprises at least 10³, at least 10⁵, at least 10⁷, at least 10⁹, at least 10¹⁰, at least 10¹¹, at least 10¹² or at least 10¹³ nucleic acids, each encoding a protein comprising an ankyrin repeat domain, and the library comprises one or more nucleic acids encoding a protein of the invention. In some embodiments, the nucleic acid library comprises at least 10³, at least 10⁵, at least 10⁷, at least 10⁹, at least 10¹⁰, at least 10¹¹, at least 10¹² or at least 10¹³ nucleic acids, each encoding a protein of the invention. In some embodiments, the nucleic acid library comprises at least 10³, at least 10⁵, at least 10⁷, at least 10⁹, at least 10¹⁰, at least 10¹¹, at least 10¹² or at least 10¹³ nucleic acids, each encoding a protein comprising an ankyrin repeat domain with a different amino acid sequence, and the library comprises one or more nucleic acids encoding a protein of the invention. In some embodiments, the nucleic acid library comprises at least 10³, at least 10⁵, at least 10⁷, at least 10⁹, at least 10¹⁰, at least 10¹¹, at least 10¹² or at least 10¹³ nucleic acids, each encoding a protein of the invention comprising an ankyrin repeat domain with a different amino acid sequence. In some embodiments, substantially all nucleic acids of the library encode a protein comprising an ankyrin repeat domain with a different amino acid sequence. In some embodiments, the nucleic acid library exclusively comprises nucleic acids encoding a protein of the invention. In some embodiments, the nucleic acid library comprises at least one nucleic acid encoding a protein of the invention.

The cell comprising the nucleic acid, the chromosome or the vector of the invention can be a prokaryotic or a eukaryotic cell. In some embodiments, the cell is a bacterial, yeast or mammalian cell. In some embodiments, the cell is derived from *E. coli, P. pastoris, S. cerevisiae,* human, hamster or mouse. In some embodiments, the cell is selected from CHO, HEK293, BHK, NS0, Sp2/0, HT-1080, PER.C6, CAP and HuH-7 cells.

In some embodiments, the *in vitro* expression system comprising the nucleic acid, chromosome or vector of the invention is based on a cell-free extract from *E. coli,* yeast, rabbit, wheat germ, insect or human.

In a further aspect, the present disclosure provides a method of preparing a protein comprising the following steps:
a) culturing a cell comprising a nucleic acid encoding the protein of the invention under conditions allowing expression thereof; and
b) purifying the expressed protein.

In one embodiment, the present disclosure provides a method of preparing a protein comprising the following steps:
a) assembling by genetic means one or more gene(s) encoding the protein of the invention, and
b) expressing the gene(s) encoding the protein of the invention.

The present disclosure also provides a pharmaceutical composition comprising the protein of the invention, the nucleic acid of the invention or the cell of the invention. In some embodiments, the pharmaceutical composition comprises an aqueous solution. For instance, it may comprise at least 1 wt% water. In some embodiments, the pharmaceutical composition is comprised in a glass or a plastic container.

In a further aspect, the present disclosure provides the use of the protein of the invention, the nucleic acid of the invention or the cell of the invention in a method of treating a disease, condition or symptom. In a further aspect, the present disclosure provides a method of treating a disease, condition or symptom comprising the administration of the protein of the invention, the nucleic acid of the invention or the cell of the invention. In a further aspect, the present disclosure provides the use of the protein of the invention, the nucleic acid of the invention or the cell of the invention in the manufacture of a medicament for the treatment of a disease, condition or symptom. In some embodiments, the disease, condition or symptom is selected from the group consisting of cancer, an immunological disease, such as an autoimmune disease, a fibrotic disease, an inflammatory disease, an ophthalmological disease, a neurodegenerative disease, an infectious disease, a nephropathy, a cardiovascular disease or a metabolic disease.

Further embodiments are as follows:
E1. A protein comprising an ankyrin repeat domain,
   wherein the internal ankyrin repeat of the ankyrin repeat domain that is adjacent to the N-terminal capping module (a) has an amino acid residue of the leucine class selected from L and I at position 23 and (b) comprises an amino acid sequence that has at least 70% sequence identity to an amino acid sequence selected from the group consisting of SEQ ID NOs: 40 to 46.
E2. The protein according to E1,
   wherein the ankyrin repeat domain has a higher melting temperature than a reference ankyrin repeat domain having the same amino acid sequence except for the amino acid residue at position 23 of the internal ankyrin repeat that is adjacent to the N-terminal capping module, which is V in the reference ankyrin repeat domain.
E3. The protein according to E1,
   wherein the N-terminal capping module of the ankyrin repeat domain (A) has an amino acid residue selected from the group consisting of I, T, A, V, L and M at position 15 and (B) comprises an amino acid sequence that has at least 70% sequence identity to an amino acid sequence selected from the group consisting of SEQ ID NOs: 1 to 37, and
   wherein, optionally, the ankyrin repeat domain has a higher melting temperature than a first reference ankyrin repeat domain having the same amino acid sequence except for the amino acid residue at position 23 of the internal ankyrin repeat that is adjacent to the N-terminal capping module, which is V in the first reference ankyrin repeat domain, and/or the ankyrin repeat domain has a higher melting temperature than a second reference ankyrin repeat domain having the same amino acid sequence except for the amino acid residue at position 15 of the N-terminal capping module, which is D in the second reference ankyrin repeat domain.
E4. The protein according to E1,
   wherein the N-terminal capping module of the ankyrin repeat domain (A) has an amino acid residue selected from the group consisting of L, V and I at position 22 and (B) comprises an amino acid sequence that has at least 70% sequence identity to an amino acid sequence selected from the group consisting of SEQ ID NOs: 1 to 37, and
   wherein, optionally, the ankyrin repeat domain has a higher melting temperature than a first reference ankyrin repeat domain having the same amino acid sequence except for the amino acid residue at position 23 of the internal ankyrin repeat that is adjacent to the N-terminal capping module, which is V in the first reference ankyrin repeat domain, and/or the ankyrin repeat domain has a higher melting temperature than a second reference ankyrin repeat domain having the same amino acid sequence except for the amino acid residue at position 22 of the N-terminal capping module, which is M in the second reference ankyrin repeat domain.
E5. The protein according to E1,
   wherein the N-terminal capping module of the ankyrin repeat domain (A) has L at position 24 and (B) comprises an amino acid sequence that has at least 70% sequence identity to an amino acid sequence selected from the group consisting of SEQ ID NOs: 1 to 37, and
   wherein, optionally, the ankyrin repeat domain has a higher melting temperature than a first reference ankyrin repeat domain having the same amino acid sequence except for the amino acid residue at position 23 of the internal ankyrin repeat that is adjacent to the N-terminal capping module, which is V in the first reference ankyrin repeat domain, and/or the ankyrin repeat domain has a higher melting temperature than a second reference ankyrin repeat domain having the same amino acid sequence except for the amino acid residue at position 24 of the N-terminal capping module, which is A in the second reference ankyrin repeat domain.
E6. The protein according to any one of E3 or E5,
   wherein the N-terminal capping module of the ankyrin repeat domain comprises an amino acid sequence that has at least 70% sequence identity to the amino acid sequence of SEQ ID NO: 35 and the internal ankyrin repeat that is adjacent to the N-terminal capping module comprises an amino acid sequence that has at least 70% sequence identity to the amino acid sequence of SEQ ID NO: 43.
E7. The protein according to any one of E1 to E6,
   wherein the N-terminal capping module of the ankyrin repeat domain does not comprise the amino acid sequence TPLH.
E8. The protein according to any one of E1 to E7, wherein said protein comprises one or more further ankyrin repeat domains.
E9. A nucleic acid comprising a sequence encoding a protein according to any one of E1 to E8.
E10. A vector or cell comprising the nucleic acid according to E9.
E11. A library of proteins comprising one or more proteins according to any one of E1 to E8.
E12. A method for selecting a protein that specifically binds to a target comprising the following steps:
   (i) providing the library of proteins according to E11; and
   (ii) selecting a protein specifically binding to the target via its ankyrin repeat domain from the library, wherein the selected protein is a protein according to any one of E1 to E8.
E13. A method of preparing a protein comprising any of the following steps (A) to (D):
   (A)
      (i) selecting a protein comprising an ankyrin repeat domain with (1) an internal ankyrin repeat that is adjacent to the N-terminal capping module that does not have L or I at position 23; and
      (ii) replacing one or more amino acid residues of the protein to result in a protein according to any one of E1 to E8;
   (B)
      (i) selecting a protein comprising an ankyrin repeat domain with (1) an internal ankyrin repeat that is adjacent to the N-terminal capping module that does not have L or I at position 23 and/or (2) an N-terminal capping module that does not have an amino acid residue selected from the group consisting of I, T, A, V, L and M at position 15; and
      (ii) replacing one or more amino acid residues of the protein to result in a protein according to any one of E3 or E6 to E8 referring back to E3;
   (C)
      (i) selecting a protein comprising an ankyrin repeat domain with (1) an internal ankyrin repeat that is adjacent to the N-terminal capping module that does not have L or I at position 23 and/or (2) an N-terminal capping module that does not have an amino acid residue selected from the group consisting of L, V and I at position 22; and
      (ii) replacing one or more amino acid residues of the protein to result in a protein according to any one of E4 or E6 to E8 referring back to E4; and
   (D)
      (i) selecting a protein comprising an ankyrin repeat domain with (1) an internal ankyrin repeat that is adjacent to the N-terminal capping module that does not have L or I at position 23 and/or (2) an N-terminal capping module that does not have L at position 24; and
      (ii) replacing one or more amino acid residues of the protein to result in a protein according to any one of E5 or E6 to E8 referring back to E5.
E14. A method of preparing a protein comprising designing or optimizing the amino acid sequence of an ankyrin repeat domain *in silico* through computational methods to result in a protein according to any one of E1 to E8.
E15. The method according to any one of E12 to E14, wherein the resulting protein is further modified with one or more further ankyrin repeat domains.
E16. A method of producing a protein comprising the following steps:
   (i) expressing or synthesizing a protein according to any one of E1 to E8 or a protein resulting from the method according to any one of E12 to E15; and
   (ii) purifying the expressed or synthesized protein.
E17. The method according to E16, further comprising the following step:
   (iii) formulating the purified protein as a pharmaceutical composition.
E18. A protein resulting from the method according to any one of E12 to E16.
E19.A pharmaceutical composition comprising any one of the following: the protein according to any one of E1 to E8 and E18, the nucleic acid according to E9 and the vector or cell according to E10,
wherein the pharmaceutical composition further comprises a pharmaceutically acceptable carrier.

### Examples

### Example 1: Effect of mutating position 23 in the internal ankyrin repeat that is adjacent to the N-terminal capping module on the thermostability of the ankyrin repeat domain

In an attempt to improve the interaction between the N-terminal capping module and the adjacent internal ankyrin repeat, various mutations were tested in the N-terminal capping module and the adjacent internal ankyrin repeat, including mutations at position 23 of the adjacent internal ankyrin repeat.

### Materials and Methods

### Protein sequences

His-tagged ankyrin repeat domains P#63 to P#65 corresponding to SEQ ID NOs: 63 to 65, respectively, were tested.

The DNA sequence encoding each ankyrin repeat domain was chemically synthesized and cloned into pQlq expression vectors (Simon M. et al., Bioconjug Chem., 23(2), 279-86, 2012) by standard techniques.

### Protein expression

The ankyrin repeat domains were expressed in *E. coli* BL21 or XL1-Blue cells and purified via their His-tag using standard protocols. Briefly, 25 ml of stationary overnight cultures (LB, 1% glucose, 100 mg/l of ampicillin; 37°C) were used to inoculate 1 l cultures (same medium). At an absorbance of about 1 at 600 nm, the cultures were induced with 0.5 mM IPTG and incubated at 37°C for 4 h. The cultures were centrifuged and the resulting pellets were resuspended in 40 ml of TBS500 (50 mM Tris-HCI, 500 mM NaCl, pH 8) and sonicated. The lysate was recentrifuged, and glycerol (10% (v/v) final concentration) and imidazole (20 mM final concentration) were added to the resulting supernatant. The ankyrin repeat domains were purified over a Ni-nitrilotriacetic acid column (2.5 ml column volume) according to the manufacturer's instructions (QIAgen, Germany). Up to 200 mg of highly soluble ankyrin repeat domains were purified from one liter of *E*. *coli* culture with a purity > 95% as estimated from SDS-15% PAGE. Such purified ankyrin repeat domains were used for further characterizations.

### CD measurement

The CD signal of the ankyrin repeat domains was recorded at 222 nm in a Chirascan V100 instrument (Applied Photophysics) while slowly heating the ankyrin repeat domains at a concentration of 0.01 mM in a buffer of PBS (137 mM NaCl, 10 mM phosphate and 2.7 mM KCl, pH 7.4) plus 2M guanidine hydrochloride (GdmCI) from 25°C to 100°C using a temperature ramp of 1°C per min, collecting data periodically at 0.5°C intervals.

Measuring the CD signal of ankyrin repeat domains is an effective means to follow their denaturation as they mainly consist of alpha helices that show a strong change in their CD signal at 222 nm upon unfolding. The midpoint of the observed transition of such a measured CD signal trace for an ankyrin repeat domain corresponds to its Tm value. Tm values were derived as described in V. Consalvi et al. (Protein Eng Des Sel. 13, 501-507, 2000).

### Results and discussion

The melting curves of ankyrin repeat domains P#63 to P#65 were determined. Based on the measured melting curves, the Tm values were determined as described above.

The influence of position 23 of the internal ankyrin repeat that is adjacent to the N-terminal capping module on thermostability of the ankyrin repeat domain was assessed by comparing P#63 to P#65 that only differ in the amino acid residue at position 23 of their internal ankyrin repeat that is adjacent to the N-terminal capping module (corresponding to position 65 of SEQ ID NOs: 63 to 65).

Figure 5 shows the melting curves of P#63 to P#65. Table 2 shows the Tm values and the corresponding amino acid residue at position 23 of the respective internal ankyrin repeat that is adjacent to the N-terminal capping module of P#63 to P#65.

**Table 2:**

| **Protein** | **Position 23** | **Tm value [°C]** |
|---|---|---|
| P#63 | V | 48.0 |
| P#64 | I | 54.9 |
| P#65 | L | 53.4 |

As reflected by Table 2, changing the amino acid residue at position 23 of the internal ankyrin repeat that is adjacent to the N-terminal capping module to an amino acid residue of the leucine class increases thermostability of the ankyrin repeat domain by up to around 7°C.

### Example 2: Effect of mutating position 23 in the internal ankyrin repeat that is adjacent to the N-terminal capping module in combination with position 15 of the N-terminal capping module on the thermostability of the ankyrin repeat domain

In order to test whether the stabilizing effect of the amino acid residue of the leucine class at position 23 of the internal ankyrin repeat that is adjacent to the N-terminal capping module was specific for the above ankyrin repeat domain or more generally applicable, ankyrin repeat domains with different binding specificities and diverging sequences were tested. Furthermore, the mutation at position 23 of the internal ankyrin repeat that is adjacent to the N-terminal capping module was tested in combination with various mutations in the N-terminal capping module, such as mutations at position 15 of the N-terminal capping module.

### Materials and Methods

### Protein sequences

His-tagged ankyrin repeat domains P#63 to P#84 corresponding to SEQ ID NOs: 63 to 84, respectively, were tested. The ankyrin repeat domains were cloned and expressed as described in Example 1.

### CD measurement

The CD measurement was done as described in Example 1, except that for measuring the CD signal of ankyrin repeat domains P#75 to P#84 4M GdmCl was used in the buffer instead of 2M GdmCl.

### Results and discussion

The melting curves of ankyrin repeat domains P#63 to P#84 were determined. Based on the measured melting curves, the Tm values were determined as described above.

The results of one set of binders having a single internal ankyrin repeat and only differing in position 15 of the N-terminal capping module and position 23 of the internal ankyrin repeat are summarized in Table 3:

**Table 3:**

| **Protein** | **Pos. 23 of the IR¹** | **Pos. 15 of the N-cap²** | **Tm value [°C]** |
|---|---|---|---|
| P#63 | V | D | 48.0 |
| P#66 | V | L | 67.6 |
| P#67 | V | V | 64.7 |
| P#68 | V | I | 65.8 |
| P#64 | I | D | 54.9 |
| P#69 | I | L | 68.2 |
| P#70 | I | V | 69.1 |
| P#71 | I | I | 69.0 |
| P#65 | L | D | 53.4 |
| P#72 | L | L | 68.4 |
| P#73 | L | V | 68.4 |
| P#74 | L | I | 69.6 |

| | | | |
|---|---|---|---|
| ¹ "IR" refers to the internal ankyrin repeat that is adjacent to the N-terminal capping module ² "N-cap" refers to the N-terminal capping module | | | |

The results of another set of binders having two internal ankyrin repeats and only differing in position 15 of the N-terminal capping module and position 23 of the internal ankyrin repeat are summarized in Table 4:

**Table 4:**

| **Protein** | **Pos. 23 of the IR¹** | **Pos. 15 of the N-cap²** | **Tm value [°C]** |
|---|---|---|---|
| P#75 | V | D | 56.5 |
| P#76 | V | L | 68.8 |
| P#77 | V | V | 66.3 |
| P#78 | V | I | 67.1 |
| P#79 | I | L | 69.4 |
| P#80 | I | V | 70.0 |
| P#81 | I | I | 70.3 |
| P#82 | L | L | 69.7 |
| P#83 | L | V | 69.3 |
| P#84 | L | I | 70.1 |

| | | | |
|---|---|---|---|
| ¹ "IR" refers to the internal ankyrin repeat that is adjacent to the N-terminal capping module ² "N-cap" refers to the N-terminal capping module | | | |

## Claims

1. A protein comprising an ankyrin repeat domain,
wherein the internal ankyrin repeat of the ankyrin repeat domain that is adjacent to the N-terminal capping module (a) has an amino acid residue of the leucine class selected from L and I at position 23 and (b) comprises an amino acid sequence that has at least 70% sequence identity to an amino acid sequence selected from the group consisting of SEQ ID NOs: 40 to 46, and
wherein the N-terminal capping module of the ankyrin repeat domain (A) has an amino acid residue selected from the group consisting of I, T, A, V, L and M at position 15 and (B) comprises an amino acid sequence that has at least 70% sequence identity to an amino acid sequence selected from the group consisting of SEQ ID NOs: 1 to 37.

2. The protein according to claim 1,
wherein the N-terminal capping module of the ankyrin repeat domain has I at position 15.

3. The protein according to claim 1 or 2, wherein said ankyrin repeat domain has a higher melting temperature than a reference ankyrin repeat domain having the same amino acid sequence except for the amino acid residue at position 23 of the internal ankyrin repeat that is adjacent to the N-terminal capping module, which is V in the reference ankyrin repeat domain.

4. The protein according to any one of claims 1 to 3, wherein said ankyrin repeat domain has a higher melting temperature than a reference ankyrin repeat domain having the same amino acid sequence except for the amino acid residue at position 15 of the N-terminal capping module, which is D in the reference ankyrin repeat domain.

5. The protein according to any one of claims 1 to 4, wherein said protein comprises one or more further ankyrin repeat domains.

6. A nucleic acid comprising a sequence encoding a protein according to any one of claims 1 to 5.

7. A vector or cell comprising the nucleic acid according to claim 6.

8. A library of proteins comprising one or more proteins according to any one of claims 1 to 5.

9. A method for selecting a protein that specifically binds to a target comprising the following steps:
(i) providing the library of proteins according to claim 8; and
(ii) selecting a protein specifically binding to the target via its ankyrin repeat domain from the library, wherein the selected protein is a protein according to any one of claims 1 to 5.

10. A method of preparing a protein comprising the following steps (A) or (B):
(A)
(i) selecting a protein comprising an ankyrin repeat domain with (1) an internal ankyrin repeat that is adjacent to the N-terminal capping module that does not have L or I at position 23 and/or (2) an N-terminal capping module that does not have an amino acid residue selected from the group consisting of I, T, A, V, L and M at position 15; and
(ii) replacing one or more amino acid residues of the protein to result in a protein according to any one of claims 1 to 5.
or
(B) designing or optimizing the amino acid sequence of an ankyrin repeat domain *in silico* through computational methods to result in a protein according to any one of claims 1 to 5.

11. The method according to claim 9 or 10, wherein one or more further ankyrin repeat proteins become linked to the resulting protein.

12. A method of producing a protein comprising the following steps:
(i) expressing or synthesizing a protein according to any one of claims 1 to 5 or a protein resulting from the method according to any one of claims 9 to 11; and
(ii) purifying the expressed or synthesized protein.

13. The method according to claim 12, further comprising the following step:
(iii) formulating the purified protein as a pharmaceutical composition.

14. A protein resulting from the method according to any one of claims 9 to 12.

15. A pharmaceutical composition comprising any one of the following: the protein according to any one of claims 1 to 5 and 14, the nucleic acid according to claim 6 and the vector or cell according to claim 7,
wherein the pharmaceutical composition further comprises a pharmaceutically acceptable carrier.
